# EUROPEAN PATENT SPECIFICATION

(11) **EP 2 788 497 B1**
(45) Date of publication and mention of the grant of the patent: **21.02.2018**
(21) Application number: 12813077.0
(22) Date of filing: 06.12.2012
(51) Int. Cl.: C12Q 1/04

(54) **MICROSENSOR**
MIKROSENSOR
MICRODÉTECTEUR

(30) Priority: 07.12.2011 GB 201120991
(43) Date of publication of application: 15.10.2014
(73) Proprietor: Microbiosensor Limited, Manchester M13 9XX (GB)
(72) Inventor: DOBSON, Curtis, Manchester M13 9PT (GB); BRAMHILL, Jane, Manchester M13 9PT (GB)
(74) Representative: Appleyard Lees IP LLP
(86) International application number: PCT/GB2012/053044
(87) International publication number: WO 2013/083993

(56) References cited:
- WO-A1-2010/099068
- WO-A2-02/30478
- US-A- 3 107 204
- US-A- 5 270 174
- D.H. KANG ET AL: "Development of a miniaturized four-culture method for the rapid enumeration of four bacterial groups in ground beef", LETTERS IN APPLIED MICROBIOLOGY, vol. 36, no. 4, 14 March 2003 (2003-03-14) , pages 197-202, XP055055439, ISSN: 0266-8254, DOI: 10.1046/j.1472-765X.2003.01273.x
- THERESA M PHILLIPS ET AL: "Colorimetric assay for Lindane dechlorination by bacteria", JOURNAL OF MICROBIOLOGICAL METHODS, vol. 47, no. 2, 1 November 2001 (2001-11-01), pages 181-188, XP055055453, ISSN: 0167-7012, DOI: 10.1016/S0167-7012(01)00299-8
- DAWIT WOLDE MESKEL ET AL: "Evaluation of a direct colorimetric assay for rapid detection of rifampicin resistant <i>Mycobacterium tuberculosis</i>", ETHIOPIAN JOURNAL OF HEALTH DEVELOPMENT, vol. 19, no. 1, 24 June 2005 (2005-06-24), XP055055461, DOI: 10.4314/ejhd.v19i1.9971
- WANG H ET AL: "An improved 3-(4,5-dimethylthiazol-2-yl)-2,5-diphenyl tetrazolium bromide (MTT) reduction assay for evaluating the viability of Escherichia coli cells", JOURNAL OF MICROBIOLOGICAL METHODS, ELSEVIER, AMSTERDAM, NL, vol. 82, no. 3, 1 September 2010 (2010-09-01), pages 330-333, XP027263333, ISSN: 0167-7012 [retrieved on 2010-07-06]

## Description

### FIELD OF THE INVENTION

The present invention provides devices and methods for detecting microorganisms in samples.

### BACKGROUND OF THE INVENTION

The rapid, reliable and accurate detection of microbial infections is a vital part of both the treatment and prevention of infection. Current methods may involve the use of liquid broths and agar and agarose based media to selectively culture microorganisms. However, one of skill will appreciate that media suitable for culturing microorganisms tend to be general purpose, permitting the growth of a broad spectrum of microorganisms or selective, permitting the growth of one or more microorganisms over others present in a sample.

General purpose media tend to become overgrown very quickly and it can be difficult to determine whether or not a sample contains a particular species or strain of microorganism. Conversely, selective media are expensive and the identification of a single species or strain in a sample is time consuming, and requires the involvement of a microbiological test laboratory, highly skilled staff, costly equipment and consumables.

Several inventions have previously been disclosed which attempt to simplify and expedite the process of detection and identification of microbes in a variety of situations. In 1973, Bucalo described a system in which culture medium held within a carrier permeable to microorganisms was inserted into a body cavity within the patient, and after inoculation and withdrawal, the contents might be subject to detailed analysis by a microbiology laboratory, to ascertain the nature of infectious agents within the patient (US3842166). The same year Freake et al. (US3881993) described a device to be dipped into a sample, and incubated in a sealable container, allowing growth, but no movement, of colonies of bacteria; metabolic indicator present in the filter layer onto which bacteria are absorbed indicated the presence of colonies. More recently, Longoria described a filtration method in which fluids diluted in inorganic acid are passed through and absorbed on a filter, before staining and destaining by the operator, revealing stained bacteria (EP0288621A).

Other approaches previously disclosed rely on the diffusion of gases from a bacterial contamination into a region of a large device sensitive to pH. Hyman Jones *et al.* describe a sensor method for detecting microorganisms (US619577B1) in which test liquids are passed through a container with a sensor plate comprising a layer for immobilising microorganisms, and a second separate layer containing indicators to detect the presence of microorganisms through local alterations in pH. Acousta et al. (WO200595635) disclose a food microbiological sensing device, in which a housing comprising a pH sensitive material with opposing first and second surfaces are held near a possible source of microbial contamination, allowing gases released by the microorganisms present in the food sample to pass through the material and cause a colour change. Alternatively a bag employing a dual colorimetric / fluorimetric sensor system, is described in WO9219764A (Morris and Green), in which after entry of clinical samples (e.g. transfusion liquids), bacteria may cause a colour change in an indicator layer, which in turn causes a change in emitted light from the fluorescent compounds, which can be detected by an optical sensor located in the bag. A simpler approach is taught in US5998161A (Caillouette) which discloses a two-step device in which test fluid is dispensed into a receiver area (which may be located on a test stick) by an operator, who then sprays or applies a reactant which changes colour in the presence of a reactant.

Sophisticated microarray technologies have also been disclosed which could provide diagnostic information regarding infectious agents, through molecular interactions of nucleic acids in clinical samples with arrays of molecular probes incorporated into test chips; such samples would be applied to these devices by a skilled operator, and detected by additional equipment or chemistry (US2002095073A1).

An improvement in the methods available for diagnosing antibiotic resistant infection has been disclosed by Frimodt-Moller, in which agar plates are divided into segments containing differing antibiotics or inhibitory agents; this can allow the identification of organisms in laboratories through recognition of particular growth patterns (WO200926920 A1).

However, none of the methods described above provide a small "micro" sensor which might be incorporated directly into a medical device, or attached discretely to a surface, allowing on-going monitoring of levels or types of microbial contamination at a specific location without any need for operator intervention. Several additional technologies might be adaptable for use in this way, however these have significantly limitations. Streckert and Tappe describe a test strip for use with diapers in which liquids pass through a membrane onto a detector layer (DE19837678A). Swanson and Rimmer describe a responsive hydrogel approach, in which a fluorochrome is incorporated into a medical device hydrogel, which is responsive to pH change caused by the presence of infectious agents (WO2008/059274). Booher (WO2006/133430) describe an apparatus for detecting microbial growth beneath a wound dressing, in which gases produced by microbial contamination pass into the device leading to change in colour of a pH sensitive dye. However all these approaches rely on a method which is not specific to microorganisms, and which requires an infection to produce a marked changed in the pH of patient tissues.

Two recent approaches involve the more microbially specific approach of use of a layer degradable by microorganisms, as the means of detection. Ferguson et al. (US2004/0043422 A1) disclose a device in which a pH sensitive / signalling layer is protected by a microbially degradable layer in contact with a patient. In the event that the device becomes colonised, the degradable layer is broken down and the signalling layer undergoes a colour change to indicate the presence of microbes. Martin et al (US 2008/0057534 A1) also make use of a microbially degradable layer, in this case uncovering a previously "hidden" clearly identifiable graphic symbol. Both these devices are capable of triggering partially, and thereby producing an unclear signal to the operator. They cannot be used to provide diagnostic information about the nature of the infection, or an indication of the numbers of infectious agents present. They are not capable of amplifying the signal produced by a challenge dose, and therefore cannot be adapted for use in situations where lower levels of infectious agents are to be detected.

Kang et al (Letters in Applied Microbiology 2003, 36, 197-202) describe the development of a miniaturized four-culture method for the rapid enumeration of four bacterial groups in ground beef.

WO02/30478 describes an indicator for the in-situ detection of the presence of a substance or a microbe at a location.

WoldeMeskel et al (Ethiop. J. Health. Dev. 2005; 19(1)) describe the evaluation of a direct colorimetric assay for rapid detection of a rifampicin resistant *Mycobacterium tuberculosis.*

Wang et al (Journal of Microbiological Methods, 82, 3, 330-333) describe an improved 3-(4,5-dimethylthiazol-2-yl)-2,5-diphenyl tetrazolium bromide (MTT) reduction assay for evaluating the viability of *Escherichia coli* cells.

US 5270174 describes a device with a large-diameter upper funnel section connected via a smaller bore (pore) to a lower compartment comprising an indicator (means for reporting) for the detection of oral bacteria. The present invention seeks to obviate one or more of the deficiencies associated with the prior art.

### SUMMARY OF THE INVENTION

This invention provides a device that can be used to analyse samples for the presence of microorganisms. In particular, the device may be used to detect the presence of specific microbial species and/or strains.
In a first aspect, the invention provides a microorganism detecting device, said device comprising a support or body component formed and adapted to have a surface for contact with a sample to be analysed, said surface defining a pore, said pore comprising means for reporting the presence of a microorganism characterised in that the reporting means comprises:
a solid or semi-solid substrate;
a tetrazolium salt; and
a nutrient broth selected from Mueller Hinton Broth or Wilkins Chalgren Broth
wherein the pore defines a volume of less than 500µl and wherein the device comprises a physical barrier that allows microorganisms to enter the reporting means but retains the components of the reporting means within the pore.

The device provided by the first aspect of this invention represents a significant improvement over the prior art as it is able to directly report the presence of microorganisms without need for additional tests or operator intervention.

It should be understood that a "sample" may be derived from any source, niche, material, substance or environment capable of harbouring microorganisms. As such, a "sample" may comprise soil (or any other natural substrate (rock, sand etc.)), food, beverages, human or animal tissue, the air (atmosphere) and/or water (fresh, marine or processed/potable). The term "sample" may include materials comprising, for example, wood, metal, plastic and/or glass. By way of example, the devices provided by this invention may be used to probe the contact surfaces (for example the surfaces of any furniture, doors, windows and/or equipment) present in domestic, commercial and/or public buildings, including, for example sports facilities, hospitals (where the devices may be used to screen surgical or ward equipment, floors, walls, ceilings and/or beds) and schools.

Sources from which samples may be derived may comprise a microbial culture (comprising one or more organisms) and the term may embrace laboratory or industrially grown microbial cultures. A sample may be derived from other sources including field sites, such as for example, an agricultural, marine, urban and/or rural site. In other embodiments, sources from which samples may be derived include human or animal subjects.

The device provided by this invention may also be used to probe or analyse "niches" or "habitats" for the presence of microorganisms. A niche or habitat may be broadly regarded as any environment capable of harbouring microorganisms and the term may include environments on or within, the human or animal body - including tissues, biological fluids, organs (for example eyes) and/or wounds. The term may further include environmental marine, urban, rural and/or agricultural niches. Again, the skilled man will appreciate that a sample for analysis using the devices described herein, may be derived from a niche or habitat.

Throughout this specification reference is made to "microorganisms" and this term should be understood as encompassing all life forms not visible to the naked eye. As such, the term "microorganism" may include, for example, bacteria, fungi, viruses, protozoa and algae. It is preferred that he devices described herein may be used to identify detect and/or quantify one or more microorganisms selected from the group consisting of, bacteria, fungi, protozoa and algae, in a sample. It should also be understood that the term "microorganism" extends to cells including, for example, mammalian, insect and/or fungal cells. As such, the devices described herein may find application in the detection of specific cell types (for example cancer cells) in samples. It is preferred that the device is used to detect bacteria and in particular pathogenic bacteria.

The devices comprise a support or body component. The support or body component may be fabricated from (or comprising) a substrate comprising or consisting of any suitable polymeric, plastic, metal, resin and/or composite material(s).One of skill will be familiar with these types of material but exemplary materials for use as substrates may include, for example, Poly(2-hydroxyethyl methacrylate) (pHEMA), Polyethylene glycol (PEG), poly(lactic-co-glycolic acid) (PLGA), Poly(methyl methacrylate) (PMMA), Polydimethylsiloxane (PDMS), Polyvinyl chloride (PVC) and/or Polyurethane (PU), polyethylene terephthalate (PET), methacrylamide derivatives such as Q9, sulphopropyl acrylates, cyclic olefins such as Topas, and/or polysulphone (PS). One of skill will readily appreciate that hydrogels comprising pHEMA may additionally include copolymers such as for example, methacrylic acid (MA), N-vinyl-2-pyrrolidone (NVC) or carboxymethyl cellulose (CMC).

The support or body component of the device may comprise a hydrophobic polymeric material. One of skill will be familiar with these types of material but exemplary materials for use may include silicone elastomers, poly(vinyl chloride), polystyrene and styrene-containing block copolymers, polyurethanes, epoxy resins, hydrophobic acrylic and methacrylic polymers with Tg>r.T. (e.g. poly(methyl methacrylate)), polycarbonates, semicrystalline or elastomeric polyolefins (e.g. ethylene-propylene rubbers, polyolefin elastomers), ethylene - vinyl acetate copolymers, polyesters such as poly(ethylene terephtalate), or polymers of lactic and/or glycolic acid, and related polyesters (e.g. poly(caprolactone), poly(2-dioxanone)).

In other embodiments, a polymeric material for use in the devices provided by this invention may comprise a densely cross-linked hydrogel. Suitable hydrogels may include those based on poly(2-hydroxyethyl methacrylate) (pHEMA).

Further exemplary materials for use in forming the support or body component of the devices provided by this invention may include other monomers in combination with HEMA, such as methacrylic acid (MA), N-vinyl-2-pyrrolidone (NVP), acrylamide (AM), N-substituted acrylamides. Other exemplary materials may include synthetic monomers other than HEMA, such as poly(ethylene glycol) (PEG) acrylated or methacrylated derivatives, or be based on polysaccharidic backbones, such as derivatives of cellulose or starch.

In a preferred embodiment the support or body component of the devices provided by this invention may comprise PET, PDMS and/or PMMA.

In one embodiment, the support or body component of the devices provided by this invention comprise poly (ethylene terephthalate) (PET).

One of skill will appreciate that a number of the materials described above possess properties which facilitate proper and prolonged functioning of the device. For example those materials which comprise a small mesh size and/or a hydrophobic nature, may be used to ensure that components of the reporting means - for example liquid components such as, for example, chromophoric compounds do not diffuse or migrate from the pore in which they are contained.

The device provided by the first aspect of this invention comprises a support or body component formed and adapted to have a surface for contact with a sample to be analysed. For convenience, the term "surface for contact with a sample" shall be referred to herein after as a "sampling surface". In one embodiment the sampling surface of the devices described herein, comprises PET.

One of skill will appreciate that the devices provided by this invention may comprise a combination of materials. For example, the device may comprise a support or body component comprising the substrate PET and one or more other suitable substrate materials. In one embodiment the sampling surface may comprise one material and the rest of the support/body component, additional or different materials. As such, the device may have a laminated or layered structure, each layer comprising a particular material.

In yet further embodiments, the device is formed and adapted to fit a sample. For example, the device may comprise a sampling surface having a profile compatible or mating with the surface of a sample to be analysed. Additionally or alternatively, the device may be flexible or deformable such that it can be shaped or formed around a sample to be analysed. In this way the devices of this invention can be brought into proper contact with a sample. In other embodiments, the sampling surface may comprise a deformable or flexible material which shapes or moulds to the surface of a sample to be analysed.

Advantageously, the sampling surface may be adapted to encourage or support the adherence, growth, colonisation and/or binding of one or more microorganisms. For example, the sampling surface may comprise one or more agents, compounds or compositions, which encourage or support microbial adherence, binding, growth and/or colonisation. It should be understood that in this context the term "comprise" encompasses the impregnation and/or coating of the sampling surface with one or more agents, compounds or compositions which support or encourage microbial growth, colonisation, adherence and/or binding. By way of example, the sampling surface may comprise one or more proteins, peptides, amino acids, carbohydrates, small organic/inorganic molecules or mixtures/complexes thereof, which either alone or together, support or encourage microbial adherence, growth, binding and/or colonisation.

One of skill may refer to a surface, coated or impregnated with a compound or composition which facilitates the colonisation, growth, adherence or binding of an entity (for example a microorganism) thereof/thereto, as a functionalised surface. As such the sampling surface of the devices described herein may be functionalised.

For convenience, the compounds or compositions, which encourage or promote microbial adherence, binding and/or colonisation shall be referred to hereinafter as "functionalising factors".

The various functionalising factors described herein may support or encourage the adherence, binding and/or colonisation of either a single species or strain of microorganism or a number of different species or strains of microorganism. Thus, the term "functionalising factors" embraces, for example growth factors, adherence factors, binding ligands and colonisation factors. The term "functionalising factors" may further encompass antibodies.

In one embodiment, the sampling surface and/or inside surface of the pore, may comprise antibodies (immobilised thereto) which exhibit and affinity, selectivity and/or specificity for one or more types of microorganism. One of skill would appreciate that the presence of microbially selective antibodies on the sampling surface or within a pore might facilitate the selective concentration of one or more specific microbes at certain regions of the device - for example at the entrance to a pore or within a pore.

The sampling surface may further comprise one or more chemoattractant compounds - said compounds serving to induce chemotaxis in microorganisms or to attract or lure any, or perhaps specific, microorganisms present in a sample towards the device and in particular the sampling surface thereof. Chemoattractant compounds/stimuli may include, for example, chemical compounds, elastic forces, electrical fields, gravity, light, magnetic fields, moving liquid, osmolality, temperature, carbohydrates (including sugars such as lactose, fructose, mannose, sorbitol, glucose, galactose, ribose or maltose), proteins (for example histine and protein kinases), amino acids (including serine, aspartate or alanine), dipeptides (including proline-leucine or glycine-proline), organic nutrients, organic acids, carbohydrates, minerals, flavones, aromatic hydrocarbons, weak organic bases such as trimethylamine, energy-linked chemicals such as oxygen, benzoate, acetate, phenol, valine, tryptophan, pyruvate, glycerol, ammonia and/or succinate. It should be understood that the sampling surface of the devices described herein may be adapted to include one or more of these chemoattractant compounds/stimuli.

Additionally, or alternatively, the sampling surface may comprise one or more components that alter the charge and/or hydrophobicity of the sampling surface. For example, the sampling surface may comprise (or further comprise) a net positive or negative charge such that negatively/positively charged microbial cells are attracted thereto.

Additionally, or alternatively, the sampling surface may comprise a hydrophobic or hydrophilic surface such that microbial cells with preferences for hydrophobic/hydrophilic surfaces are attracted thereto. In this regard, the sampling surface of the device may comprise poly(propylene) (PP), poly(tetrafluoroethylene) (PTFE), poly(urethane) PU, poly(vinylchloride) (PVC), poly(ethyleneoxide-co-propyleneoxide) (A-B PEO-co-PPO), poly(ethyleneglycol) (PEG), poly(styrene sulfonic acid sodium salt) (PSSNa), potassium salt of 3-sulfopropyl acrylate (KSPA), sodium 2-acrylamido 2-methyl propane sulfonic acid (NaAMPs), poly(diallydimethylammonium chloride) (PDADMAC).

In other embodiments, the sampling surface may comprise one or more compounds known to be involved in quorum sensing in microbial organisms. One of skill will appreciate that in response to certain compounds, microorganisms can adopt certain behaviour. For example, quorum sensing compounds may induce swarming or motility. Quorum sensing is particularly prevalent in species of *Vibrio, Escherichia, Salmonella, Pseudomonas, Acinetobacter* and *Aeromonas.*

It should be understood that chemoattractant and/or quorum sensing compounds are to be regarded as functionalising factors.

In one embodiment, the sampling surface comprises one or more plasma treated areas - the plasma treated areas being regarded as functionalised.

In view of the above, one embodiment of this invention provides a microorganism detecting device comprising a support or body component formed and adapted to have a plasma treated surface for contact with a sample to be analysed, said surface defining a pore, said pore comprising means for reporting the presence of a microorganism.

In one embodiment, the devices described herein comprise a sampling surface which has been subjected to treatment with oxygen and/or nitrogen plasma. One of skill will appreciate that treatment with oxygen plasma creates a hydrophilic surface via the modification of the polymer surface to generate C-O, C=O and/or O=C-O groups. Treatment with nitrogen plasma is an alternative which improves surface compatibility with proteins and DNA.

Advantageously, the sampling surface may comprise one or more discrete, distinct or predetermined locations or regions, each location or region comprising one or more functionalising factors. In one embodiment, different functionalising factors may be present at each of the distinct or discrete locations or regions of the sampling surface. In a yet further embodiment, the functionalising factors described herein may be applied to, or arranged on or within, the sampling surface so as to form an array of discrete or distinct spots, each comprising one or more functionalising factors. It should be understood that the spaces in-between each of the distinct or discrete locations/regions of the sampling surface may be devoid of functionalising factors or may comprise one or more factors known to inhibit or prevent microbial growth ("inhibitory factors"). Again, these inhibitory factors may be coated on, or impregnate into, the sampling surface and may comprise, for example, antibiotics, antifungals, antivirals, cytotoxic agents and the like.

One of skill will appreciate that by providing a sampling surface which comprises one or more functionalising factors within one or more discrete or distinct locations or regions of the sampling surface, it should be possible to encourage microorganisms to colonise or bind or adhere to, one or more predetermined, targeted or specific locations or regions of the sampling surface. Furthermore, by ensuring the device comprises different functionalising factors in different regions or locations of the sampling surface, it may be possible to ensure that different microorganisms colonise or bind or adhere to, predetermined, select or targeted regions of the sampling surface.

The device provided by the first aspect of this invention may comprise a sampling surface defining a single pore, or a plurality of (i.e. two or more) pores.

In one embodiment, the pore or pores described herein is/are open to the surface of the sampling surface and extend into the support or body component of the device. The pores may be blind ended or, in other embodiments, they may extend right through the substrate.

The pores defined by the sampling surface are wide enough to allow the passage of microorganisms and/or cells, but narrow enough to exclude non-microbial life forms. In this way, the pores may impart a selectivity to the device - acting as "size" base filters. In one embodiment, the pores defined by the sampling surface are each approximately 0.5µm -2000µm wide. In one embodiment, the pores may be between about 1µm and about 1500µm or 1000 µm wide. In other embodiments, the pores may be about 11µm, 2µm, 3µm, 4µm, 5µm, 6µm, 7µm, 8µm, 9µm, 10µm, 20µm, 30µm, 40µm, 50µm, 70µm, 100µm, 200µm, 300µm, 400µm, 500µm, 600µm, 700µm, 800µm or 900µm wide. In one embodiment, the pore or pores may be about 500µm wide. In some embodiments the pores may be about 1mm and 3mm wide. The pores may be about 1mm, 1.2mm, 1.4mm, 1.6mm, 1.8mm, 2mm, 2.2mm, 2.5mm, 3mm, 4mm or 5mm wide. One of skill will appreciate that each of the pores defined by the sampling surface may be the same width or they may have different widths.

Microorganisms come in many different shapes and sizes and by varying the width of the pores defined by the sampling surface, it may be possible to make the pores "selective" or "receptive" to certain types of microbial species. For example, large microorganisms or organisms that are not microscopic, will not be able to enter small pores. In one embodiment, the pores may act as filters, allowing passage of microorganisms to the reporting means, but preventing passage of larger organisms. Indeed, by ensuring the pores defined by the sampling surface of a particular width, it may be possible to make them selective to the passage of microorganisms in general or to specific microorganisms.

In one embodiment, the pores are between about 0.5µm and about 1000µm long. The pores may be about 1µm, 2µm, 3µm, 4µm, 5µm, 6µm, 7µm, 8µm, 9µm, 10µm, 20µm, 30µm, 40µm, 50µm, 60µm, 70µm, 80µm, 90µm, 100µm, 150µm, 200µm, 300µm, 400µm, 500µm, 750µm or about 900µm long. In other embodiments, the pores may be between about 1mm and 20mm long. The pores may be about 1mm, 2mm, 5mm, 10mm, 15mm, 20mm, 25mm or about 30mm long. In some embodiments, the pores may be 10, 20, 50, 100, 200 or 300 cm long. One of skill will appreciate that each of the pores defined by the sampling surface may be the same length or they may have different lengths.

In one embodiment, the pores may be about 20µm wide and about 50µm long, 500µm wide and about 250µm long, 250µm wide and about 500µm long.

It is to be understood that the pores defined by the sampling surface of the device described herein, may comprise any combination of the dimensions described herein - i.e. any width with any length. Further, it should be understood that the precise width and depth of any pore defined by the sampling surface of the devices described herein is to be calculated on the basis of the size of microorganism to be excluded from the reporting means (or rather the size of the microorganisms to be permitted passage through the pore(s) and into contact with the reporting means). As such, while we have attempted to provide the reader an indication of the likely dimensions of any pore to be defined by the sampling surface of the devices of this invention, the pores should be formed and adapted to permit passage of a predetermined range or type of microorganism.

The sensitivity of the devices described herein may be altered by adjusting the dimensions of the pore(s). For example a device having high sensitivity, i.e. reporting the presence of low levels of microbial contamination within a sample may comprise one or more wide pores of short length - in one embodiment, the pores may be wider than they are long. Conversely, a low sensitivity device may be made with narrow and long pores - i.e. pores which are longer than they are wide.

In one embodiment, the opening of each pore defined by the sampling surface may comprise a protective cover - the protective cover preventing dust and other particulate matter from falling into or entering the pore.

In one embodiment, the part or parts of the sampling surface that define the pore openings comprise one or more functionalising factors. For example, functionalising factors may be coated to, or impregnated within, the sampling surface, around (or in the vicinity of) the opening of each pore defined therein. In this way, microorganisms can be encouraged to colonise or adhere or bind to those parts of the sampling surface that define pore openings. In other embodiment, the surface of the inside of the pore (the interior surface or the bore or pore shaft) may further comprise (i.e. be coated or impregnated with) functionalising factors.

Thus, a further embodiment of this invention provides a microorganism detecting device comprising a support or body component formed and adapted to have a surface for contact with a sample to be analysed, said surface comprising one or more discrete locations, each location comprising one or more functionalising factors and defining a pore, said pore comprising means for reporting the presence of a microorganism.

In one embodiment the part of the sampling surface that defines the opening of the pore(s) comprises a depression, recess or dimple which may, for example, have a concave or tapered profile/cross-section. Typically, the opening of the pore is located at the base or bottom (or lowest point) of the depression, recess or dimple, which depression, recess or dimple then tapers out or extends from the pore opening towards, the outer surface of the sampling surface. In one embodiment, the depression, recess or dimple may take the form of a countersink and the opening of each pore defined by the sampling surface may be countersunk below the sampling surface.

In use, the depression, recess or dimple may act as a funnel guiding microorganisms present on the sampling surface down and into the pore(s) defined therein.

The presence of a dimple, recess or other type of depression at the sampling surface of the device may further increase the surface area of the sampling surface and, without wishing to be bound by any particular theory, may increase the area of the sampling surface upon which microorganisms present in the sample being analysed adhere.

In one embodiment, the sampling surface may further comprise a textured or profiled surface so as to further increase the area upon which a microorganism may adhere and/or grow.

In one embodiment, the surface of each of the depressions, recesses, dimples or countersinks present in the sampling surface comprise one or more functionalising factors. In other embodiments, the spaces between each of the depressions, recesses, dimples or countersinks present in the sampling surface are devoid of functionalising factor or comprise inhibitory factors as described above.

As such, a further embodiment of this invention provides a microorganism detecting device comprising a support or body formed and adapted to have a surface for contact with a sample to be analysed, said surface comprising one or more discrete functionalised countersinks, the bottom of each countersink defining a pore comprising means for reporting the presence of a microorganism.

The means of reporting the presence of a microorganism may be adapted to report the presence of (i) a specific species or strain of microorganism or (ii) a number of different species and/or strains of microorganisms.

The means for reporting the presence of a microorganism shall be referred to hereinafter as a "reporting means".

The reporting means may be contained within each of the pores defined by the sampling surface of the device provided by this invention. In one embodiment, the reporting means may be contained at the end of the pore. In other embodiments, one or more of the pores defined by the sampling surface may connect to a chamber containing a reporting means. For example, the reporting means may be contained within a chamber, for example a bulbous chamber, located at the end (or bottom) of the pore; in such cases the pore may terminate with or connect to said chamber. In other embodiments, the reporting means may be contained within a chamber defined by the support or body component of the device. In such cases, the one or more pores defined by the sampling surface of the device would connect to the one or more chambers defined by the support or body component. It should be understood that the device may comprise a single chamber comprising a reporting means or a plurality of chambers each comprising a reporting means. A pore defined by the support or body component of the device may extend into the substrate of the support body component to connect with one or more chambers, at least one of which may comprise a reporting means. Where each pore connects to a plurality of chambers - each chamber may comprise a reporting means. Where there is a plurality of pores, each pore may connect to a chamber defining a reporting means. Alternatively, a plurality of pores defined by the support or body component of the device may extend into the substrate and connect with a reporting means (or chamber containing the reporting means). As such, while each pore may connect to a reporting means (or chamber containing the same), more than one pore defined by the support or body component of the device may connect to a single reporting means (or chamber containing the same).

In one embodiment, the chamber comprising, housing or containing the reporting means may be considerably larger than the pore connecting to it. For example, the chamber may be approximately 1-5mm in width/diameter and 1-5mm in depth. The chamber may define a spherical, or cylindrical chamber. In one embodiment, the chamber may be about 2mm deep and about 1mm wide. The precise size of the chamber may not be crucial however it should be sufficiently large to contain all the components required to report the presence of a microorganism in a sample at the required level of sensitivity.

Additionally, the device may comprise a "containment" layer - located above the reporting means or between the pore and any chamber comprising, housing or containing the reporting means. The layer may be adapted to allow microorganisms to enter the reporting means (or chamber(s) containing the same) but substantially retain the components of the reporting means within any chamber housing or containing the same. In one embodiment the barrier/containment layer may prevent any material contained within the reporting means (or chamber(s)), for example solutions and the like, from desiccation and/or diffusion of low molecular weight constituents.

In one embodiment, the containment layer may comprise a viscous liquid (which may be hydrophobic or hydrophilic) approximately 1 - 1000 µm thick or deep between the pore and the reporting means. In preferred embodiments, the barrier/containment layer is approximately 100-400µm thick/deep. The containment layer may be alginate based, pectin based, hyaluronic acid, glycerol or cellulose based. In a preferred embodiment the containment layer is carboxymethyl cellulose.

The device comprises a physical barrier that allows microorganisms to enter the reporting means but retains the components of the reporting means within the pore. Preferred barriers are meshes with a mesh pore size that is large enough to allow bacteria into the reporting means but small enough to prevent dislodgement of the reporting means and ingress of larger cells (e.g. mammalian cells) or particles. Preferred meshes may have a mesh pore size of 1-1,000µm, preferably a mesh pore size of 50-500µm, more preferably a mesh pore size of 75-200µm and most preferably a mesh pore size of approximately 100µm. Typically a mesh will be less than 500µm thick. Meshes may be manufactured from PMMA, PET or polypropylene and functionally equivalent polymers. A most preferred mesh has a mesh pore size of approximately 100µm and may be fabricated from 150µm thick PMMA.

In one embodiment, the device may comprise a fixing agent (for example a gum) to prevent the egress of microorganisms from the chamber comprising the reporting means.

The reporting may comprises a solid or semi-solid substrates. One of skill will appreciate that a solid or semi-solid substrate may comprise a quantity of agar or agarose - the precise quantity being determined by the degree of substrate solidity required. It is preferred that the solid or semi-solid substrate is agar or agarose. By way of example, the reporting means may comprise 0.1%-1.5% w/v agar mix, preferably 0.75%-1.4% w/v agar mix, more preferably 0.9%-1.1% w/v agar mix and most preferably about 1% w/v agar mix. Insofar as agarose is concerned, the reporting means may comprise 0.3%-1.0% w/v agarose mix, preferably 0.5%-0.9% w/v agarose mix, more preferably 0.6%-0.8% w/v agarose mix and most preferably about 0.7% w/v agarose mix.

The reporting means comprises a tetrazolium salt. It is preferred that the reporting means comprises a tetrazolium salt such as MTT (3-(4,5-Dimethylthiazol-2-yl)-2,5-diphenyltetrazolium bromide). One of skill will appreciate that the yellow MTT is reduced to purple formazan in living cells. Suitable additional or alternative indicators for use in the reporting means of any of the devices provided by this invention may include, for example, XTT (2,3-bis-(2-methoxy-4-nitro-5-sulfophenyl)-2H-tetrazolium-5-carboxanilide), MTS (3-(4,5-dimethylthiazol-2-yl)-5-(3-carboxymethoxyphenyl)-2-(4-sulfophenyl)-2H-tetrazolium) or water soluble tetrazolium salts (WST) such as WST-1, WST-3, WST-4, WST-5, WST-7, WST-8, WST-9, WST-10 or WST-11. Alternatively, other tetrazolium salts may be used including indonitrotetrazolium chloride (INT), Nitrobluetetrazolium (NBT), Tetranitro blue tetrazolium (TNBT), Thiocarbamyl nitro blue tetrazolium (TCNBT) or Tetrazolium red (TR).

Embodiments of this invention in which MTT is exploited in the reporting means, may comprise at least about 10µg/ml, 20µg/ml, 30µg/ml, 40µg/ml, 50µg/ml, 75µg/ml, 100µg/ml, 125µg/ml, 150µg/ml, 200µg/ml, 250µg/ml, 300µg/ml, 350µg/ml, 400µg/ml, 450µg/ml, 500µg/ml, 550µg/ml, 600µg/ml, 750µg/ml or about 1,000µg/ml MTT. Preferably the reporting means comprises 100-750µg/ml and more preferably about 400-600µg/ml of MTT. In a preferred embodiment about 500µg/ml of MTT is used. About the same quantities of MTS may be employed when it is used instead of MTT.

Additionally, the reporting means may comprise a pH indicator solution such as toluylene (Neutral) red, which changes from red to yellow at a pH of about 6.8-8. pH indicator solutions are particularly useful for the detection of microorganisms which produce metabolites having a pH which differs (or contrasts with) the pH of the local environment. For example, the a pH indictor such as neutral red may be added to the reporting means which has an overall acidic, neutral or alkali pH. Any production of a metabolite having a pH different from that of the reporting means will affect a colour change in the pH indicator solution.

It will be appreciated that the reporting means may comprise a chemiluminescent, bioluminescent or fluorescent indicator.

A significant advantage of many devices according to the invention is that they may be read 'by eye'. However under some circumstances (e.g. when a chemiluminescent, bioluminescent or fluorescent indicator is used or when very faint colour changes may occur) it may be desirable to 'read' the reporting means by something other than a human eye. Therefore in one embodiment a scanner or detector may be used. In this case it may be desirable to incorporate a 'label' reader (e.g. a barcode) to simplify the process of indicating when a particular sensor had been examined. For instance a nurse may scan a microsensor incorporated in a medical device on a hospital ward to see if it triggered. The scanner would record the barcode and result to indicate on the patient record what has been done.

In one embodiment, the reporting means may comprise one or more substances to be tested against one or more microorganisms. For example, the reporting means may comprise one or more substances against which microbial sensitivity is assessed. Such substances may be referred to as "test agents" and may include - antibiotics and/or drug candidate compounds. One of skill will appreciate that where, for example, the test agent is an antibiotic, the device may be used to determine whether or not a particular microorganism is sensitive thereto. In one embodiment, and where a plurality of chambers is connected to a pore, each chamber may comprise a different test agent (and/or a different concentration of the same test agent).

In one embodiment, the reporting means of the devices described in this invention may comprise 1.5x Mueller-Hinton broth (a "growth factor").

One of skill will appreciate that by combining one or more growth factors into or with the reporting means, the reporting means may encourage, support and/or enrich the growth of one or more microorganisms while at the same time reporting (perhaps via one or more of the indicator components described herein) the presence of one or more of the microorganisms growing therein.

One of skill will appreciate that by incorporating growth factors into the device described herein - either as a coating or impregnation of the sampling surface or pore or as a component of the reporting means, it is possible to detect very low numbers of microorganism present in a sample. In effect, the growth factors present in the devices described herein, amplify the number of microorganisms and may make the device sensitive to very low numbers of microorganisms in samples. Prior art devices lack growth factors and as such are much less sensitive and may need to be left *in situ* for extended periods of time in order to achieve positive detection. When the sample is a wound, for example, this is undesirable as the prolonged contact between a microbial detection device and the wound significantly increases the likelihood of infection.

In one embodiment, the device provided by this invention may further comprise one or more pump elements to move fluid components (for example components of the reporting means). By way of example, components of the reporting means, for example, indicators and/or growth factors, may be provided in a reservoir connected to the device (and in particular one or more of the pores thereof) via a pump, for example a peristaltic pump. In this way, expired growth factors and/or indicator means in the reporting means could be replenished. In other embodiments, pumps, for example, peristaltic pumps, may be exploited to effect the movement of fluid components of the reporting means - including growth factors and growth media, over microorganisms present in the chamber containing the reporting means of within the pore.

The device provided by this invention may be contacted with the sample and incubated therewith, perhaps only for a brief or for a very short period of time (for example seconds, minutes, hours or days) - the growth factors present in the device serving to enhance the growth of any microorganisms transferred to the sampling surface during the incubation period and ensuring positive detection.

Alternatively, the device (or the sampling surface thereof) could be inoculated using a swab taken from a sample (for example a wound or other surface). In such cases, the device need never be directly contacted with the sample being analysed - this of course, substantially reduces the risk of infection in wounds. Again, even though only a very small number of microorganisms might be transferred to the sampling surface of the device by a swab, any growth factors present on or within the sampling surface, pore and/or reporting means, would serve to amplify the number of microorganisms.

In one embodiment, the devices provided by this invention may be exploited as microsensors. The microsensors may be formed and adapted to be microsensors exhibiting a high or low degree of sensitivity to microorganisms present in samples. As stated above, the degree of sensitivity exhibited by devices of this invention may be modulated by altering one or more of the pore size and number and/or the type quantity and/or location of growth factors within the device (for example, growth factors may be present on the sampling surface, through the pores and/or as part of the reporting means.

In a further embodiment, the devices described herein may be provided as a component of a further device, apparatus or material. For example, the devices described herein might be used to report microbial contamination of a secondary device, apparatus or material. In one embodiment, the colour change or other reaction occurring in the reporting means to indicate the presence of a microorganism, is assessed by a separate optical detection device. On triggering this apparatus might alert an operator remotely so that appropriate action might be taken. Such apparatus may also provide for automatic removal and replacement of devices after predetermined time intervals, such that the device was available to be triggered for specific time periods.

One application may be in the field of air conditioning where parts of the device and/or water contained therein, may be analysed or probed for the presence of microorganisms. A further application may be in the field of medical devices, including contact lenses and contact lens cases, wound dressings, intravenous and urinary catheters, endotracheal tubes, dental and orthopaedic implants, intraocular implants and surgical equipment that must be kept sterile. By incorporating a device according to this invention into a contact lens case, contact lens or sterile dressing or other medical device it may be possible to detect any microbial contamination thereof. Other applications of the devices provided by this invention include, for example, incorporation into food packaging as a means to monitor the sterility of food or veterinary applications including incorporation into bandages, casts or dressings a means to monitor microbial contamination of wounds etc.

Many microorganisms, particularly bacteria, fungi and/or protozoa, are motile and can move or propel themselves through substrates and across surfaces. Non-motile organisms may be static and may remain bound or adhered to a surface; however, by replicating, non-motile microorganisms may colonise large areas in a relatively short period of time. In this way a developing colony of one or more microorganisms may spread across a surface or from one surface to another.

One of skill will appreciate that microorganisms present in a sample being analysed may move towards or colonise the device (or sampling surface thereof) provided by this invention. Advantageously, the presence of functionalising factors on or within the sampling surface may facilitate, promote or encourage the migration of microorganisms (either through motility or colonisation spread) to the sampling surface of the device. Further, the presence of adherence factors (as functionalising factors) on or within the sampling surface, may promote or increase the adherence of microorganisms thereto. Moreover, by restricting the adherence factors (and other functionalising factors) to the (immediate) area surrounding the pore openings defined by the sampling surface, it may be possible to ensure that any microorganisms passing from the sample being analysed, colonise or bind or adhere to those areas defining the pore openings. The targeting of microbial adherence/colonisation to select or predetermined areas of the sampling surface, can be enhanced by the selective placement of inhibitory factors in areas where microbial growth is not desired.

One of skill will further appreciate that where the device comprises a sampling surface having one or more countersinks, depressions, dimples or recesses, the positioning of the pore opening at the base or bottom of any such formation and the functionalisation of the surface thereof, might facilitate the passage of microorganisms towards the pore opening. The shape of the depression or dimple which surrounds the opening of the pore(s) (i.e. tapered or concave in profile or cross-section) may act as a funnel to channel microorganisms present in the sample towards the opening of each of the pores defined by the sampling surface.

Thereafter, the microorganism may move down (or colonise the surface of) the pore, making their way towards the reporting means. In one embodiment, the functionalising factors may be applied to the inside surface of the pore.

When brought into contact with the reporting means, any growth factors present in the reporting means will induce further microbial growth and any indicator present will report the presence of microbial pathways and/or metabolites as described above.

This invention provides an elegant, inexpensive technology providing real-time, clear and simply understood information to an untrained operator, untrained wearer of a medical device, or untrained user of an area being monitored for microbial contamination. Variants of the device can provide real-time diagnostic information to skilled operators, without the need for operator intervention or sample processing. Uniquely the combination of a selective pore and a reporting means that facilitates bacterial growth within the device allows the safe, limited and controlled growth of challenge organisms, thereby enabling control of threshold (such that a low, medium or high level of infection can be detected), and giving rise to the same clear all or nothing response, once the trigger dose has been reached. Additionally, by combining several or many microsensors containing differing types of medium, antibiotic or inhibitor, detailed diagnostic information can be conveyed to the physician or operator, enabling immediate intervention at the early stages of infection. Importantly the physician can quickly be alerted to the correct type of antibiotic to treat the patient with, thereby ensuring effective treatment, and avoiding the overuse of antibiotics unnecessarily. Similarly the rapid clear output of the device enables early intervention for environmental monitoring applications. The device produces its signal without any operator involvement. If incorporated within medical devices, the medical device manufacturer will select variants of the microsensor, and sufficient numbers of the microsensor such that useful early clinical information can be provided during normal use of the device, without any additional operations or procedures by patient or healthcare staff being required.

The devices according to the first aspect of the invention have a reporting means which comprises: a solid or semi-solid substrate; a metabolic indicator for reporting the presence of living organisms or cells, which is a tetrazolium salt; and a media that supports or encourages microbial growth, which is either Mueller Hinton Broth or Wilkins Chalgren Broth. The inventors have surprisingly found that such reporting means:
(a) provide real-time, clear and simply understood information on microbial contamination to an untrained operator, untrained wearer of a medical device, or untrained user of an area being monitored for microbial contamination; and
(b) can be incorporated in small volumes into miniature devices according to the invention (also referred to herein as "microsensors") while retaining suitable sensitivity and selectivity for reporting microbial contamination.

It is preferred that the solid or semi-solid substrate is agar or agarose. The precise quantity being determined by the degree of substrate solidity required. By way of example, the reporting means may comprise 0.1%-1.5% w/v agar mix, preferably 0.75%-1.4% w/v agar mix, more preferably 0.9%-1.1% w/v agar mix and most preferably about 1% w/v agar mix. Insofar as agarose is concerned, the reporting means may comprise 0.3%-1.0% w/v agarose mix, preferably 0.5%-0.9% w/v agarose mix, more preferably 0.6%-0.8% w/v agarose mix and most preferably about 0.7% w/v agarose mix.

The tetrazolium salt may be MTT (3-(4,5-Dimethylthiazol-2-yl)-2,5-diphenyltetrazolium bromide), XTT (2,3-bis-(2-methoxy-4-nitro-5-sulfophenyl)-2H-tetrazolium-5-carboxanilide), MTS (3-(4,5-dimethylthiazol-2-yl)-5-(3-carboxymethoxyphenyl)-2-(4-sulfophenyl)-2H-tetrazolium) or water soluble tetrazolium salts (WST) such as WST-1, WST-3, WST-4, WST-5, WST-7, WST-8, WST-9, WST-10 or WST-11. Alternatively, other tetrazolium salts may be used including indonitrotetrazolium chloride (INT), Nitrobluetetrazolium (NBT), Tetranitro blue tetrazolium (TNBT), Thiocarbamyl nitro blue tetrazolium (TCNBT) or Tetrazolium red (TR).

It is most preferred that MTT or MTS are used.

The reporting means, may comprise at least about 10µg/ml, 20µg/ml, 30µg/ml, 40µg/ml, 50µg/ml, 75µg/ml, 100µg/ml, 125µg/ml, 150µg/ml, 200µg/ml, 250µg/ml, 300µg/ml, 350µg/ml, 400µg/ml, 450µg/ml, 500µg/ml, 550µg/ml, 600µg/ml, 750µg/ml or about 1,000µg/ml of a tetrazolium salt. Preferably 100-750µg/ml and more preferably about 400-600µg/ml are used. In a preferred embodiment about 500µg/ml of MTT is used. About the same quantities of MTS may be employed when it is used instead of MTT.

The inventors have found that a number of factors can potentially lead to the degradation of the reporting means or can lead to the false triggering (i.e. the generation of the reporting signal in the absence of a micro-organism). The inventors do not wish to be bound by any hypothesis but they believe that such false triggering can be caused by:
(a) steps in the manufacture of preferred devices according to the invention (e.g. an autoclaving step); and
(b) inappropriate selection of the media and/or nutrients that support or encourage microbial growth.

Insofar as (a) is concerned, and by way of example, the inventors have noted that tetrazolium salts may be converted to formazan by the action of heat under some circumstances. They have therefore realized that the manufacturing process for devices according to the invention should not include a step in which such indicators are heated. For instance, in the preparation of the reporting means the substrate and media and/or nutrients may be combined and autoclaved to make them sterile. However a tetrazolium salt is ideally made-up in solution and filtered to make it sterile and then only combined with the other elements of the reporting means after the autoclave step. However it will be appreciated that there are circumstances (e.g. where reducing agents can be precluded from solution - for instance MTT dissolved in distilled water) where heating may not be an issue for tetrazolium salts.

Insofar as (b) is concerned, a media and/or nutrients should be chosen that:
(i) maintains viable micro-organisms in the pore and in some embodiments supports or encourages microbial growth and/or division in the pore;
(ii) takes into account the extent that the viability of a specific species, narrow spectrum or broad spectrum of micro-organisms needs to be maintained and detected;
(iii) will not result in false triggering of the indicator in the absence of micro-organisms; and
(iv) preferably is capable of permitting selective and sensitive sensing of micro-organisms in volumes of reporting means that are less than 500µl and more preferably less than about 50µl.

The inventors applied a great deal of inventive endeavour to select media and/or nutrients with the correct balance of features (i)-(iv) above. When tetrazolium salts are used, the inventors found that these indicators are inappropriately converted to formazan by a number of commonly used media/broths used for growing micro-organisms bacteria without being exposed to the micro-organisms (i.e. (iii) above was at issue). They were able to assay this by measuring, over time, the Optical Density (at 570nm) of media solutions mixed with the tetrazolium salts. Media/broths were discounted that turned black (i.e. formazan was formed) when the broth containing the indicator was incubated overnight (i.e. 10-20 hours) at 40°C. Accordingly the media and/or nutrients used in the reporting means of the first aspects of the invention is Mueller Hinton and Wilkins Chalgren broths.

It is preferred that pores of devices according the first aspect of the invention which are cylindrical have a diameter of less than 10mm and preferably a diameter of less than 5mm. Preferred cylindrical pores have a diameter of between 0.5 and 4mm and may have a diameter of 4, 2 or 1mm or 0.5mm. It will be appreciated that it is preferred that, for each of use, that the pore is not so small that it will be invisible to the naked eye. Preferred cylindrical pores are less than 10mm deep, more preferably less than 5mm deep and preferably about 2mm deep or less. Preferred cylindrical pores may be 4x2mm, 2x2mm or 1x1mm (diameter x depth).

A significant advantage of the devices according to the invention is that the inventors have been able to devise miniaturised devices (i.e "microsensors") that have applications that could not be envisaged for the microbial sensors that are known to the art that tend to be large and/or require a skilled user. This miniaturization has only been possible by developing pores with a limited volume. Significant inventor endeavour has been employed to develop small devices which comprise suitably sensitive and selective reporting means. Devices according to the invention may typically have pores which are a few millimetres deep or even only a few micrometres deep. Accordingly the pore defines a volume of less than 500µl, less than 250µl, less than 100µl, less than 50µl or less than 5µl. In one embodiment of the invention, a pore may be portioned such that it will have a volume of about 25µl which may be adapted to contain about 20 µl of a reporting means and about 5µl of a liquid containment layer (see below). In another preferred embodiment a pore may be portioned such that it will have a volume of about 6.2µl which may be adapted to contain about 5 µl of a reporting means and about 1.2µl of a liquid containment layer. In another preferred embodiment a pore may be portioned such that it will have a volume of about 1.6µl which may be adapted to contain about 1.4 µl of a reporting means and about 0.2µl of a liquid containment layer.

The device of the invention may also comprise a "containment" layer as previously discussed. The containment layer may be located between where the sample will be located when in use and the reporting means in the pore. The layer should be adapted such as to allow microorganisms to enter the reporting means but substantially retain the components of the reporting means within the pore. The layer may be effective for maintaining the viability of the reporting means such that the device will have a shelf-life of several weeks, several months or more preferably a shelf-life of one, two or more years.

The containment layer is preferably contained within the pore and lies over the top of the reporting means. In some embodiments the pore may be contained within a depression, recess or dimple in the sampling surface of the device as discussed in connection with the first aspect of the invention. In such embodiments the containment layer may be positioned in the depression, recess or dimple above the pore.

In one embodiment the containment layer may prevent any material contained within the reporting means becoming desiccated and/or prevent diffusion of low molecular weight constituents out of the reporting means. The containment layer may comprise a viscous liquid approximately 1 - 1,000 µm thick or deep between where the sample will be located when in use and the reporting means. In preferred embodiments, where a cylindrical pore is 1-2mm deep the barrier/containment layer will typically be 100-400µm thick/deep. The containment layer may be alginate based, pectin based, hyaluronic acid, glycerol or cellulose based. In a preferred embodiment the containment layer is carboxymethyl cellulose. By way of example the inventors have found that 5µl of 5% carboxymethyl cellulose in PBS represents an effective containment layer for a 4x2mm cylindrical pore with a volume of about 25µl. The remaining 20µl of such a pore may comprise the reporting means (e.g. 20µl of a Wilkins Chalgren Agar containing 500µg/ml MTT).

Devices according to the first aspect of the invention include a physical barrier that acts to retain the reporting means within the pore. Such a physical barrier may be located between where the sample will be located when in use and the reporting means. Alternatively the barrier may be between where the sample will be located when in use and the containment layer (if present) or the barrier may be between the containment layer and the reporting means.

The physical barrier may be contained within the pore and lie over the top of the reporting means. In preferred embodiments the pore may be contained within a depression, recess or dimple in the sampling surface of the device as discussed in connection with the first aspect of the invention. In this case it is preferred that the reporting means and containment layer are contained within the pore and the physical barrier is positioned over the pore and fixed within the depression, recess or dimple.

The physical barrier is adapted such as to allow microorganisms to enter the reporting means but retains the components of the reporting means within the pore. Preferred barriers are meshes with a mesh pore size that is large enough to allow bacteria into the reporting means but small enough to prevent dislodgement of the reporting means and ingress of larger cells (e.g. mammalian cells) or particles. Preferred meshes may have a mesh pore size of 1-1,000µm, preferably a mesh pore size of 50-500µm, more preferably a mesh pore size of 75-200µm and most preferably a mesh pore size of approximately 100µm. Typically a mesh will be less than 500µm thick. Meshes may be manufactured from PMMA, PET or polypropylene and functionally equivalent polymers. A most preferred mesh has a mesh size of approximately 100µm and may be fabricated from 150µm thick PMMA.

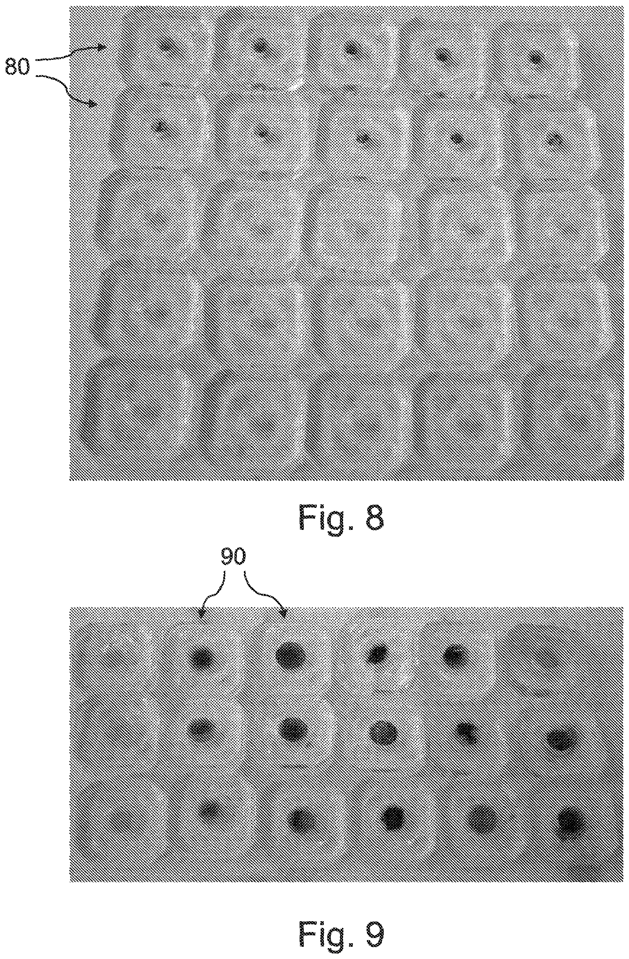

Devices according to the invention may be independent devices which essentially comprise a housing for the pore (and the reporting means contained therein) and any containment layer or physical barrier. The housing is preferably formed to enable or encourage microorganisms to enter the pore. The housing may be adapted as discussed above in connection with the first aspect of the invention. Examples of such a device are shown in figures 8 and 9.

Other devices according to the invention may be adapted to fit to, or even be an integral part of, other devices. Devices according to the invention that have been incorporated in this way may be used to detect micro-organisms within samples contained within such other devices or to detect micro-organisms to which such other devices are exposed. Devices according to the invention may be fitted inside a variety of medical devices - in fact any other device where it is relevant to monitor for microbial contamination. In a preferred emboidment, and as discussed in more detail below, a device(s) according to the invention may be incorporated within another device which is a contact lens holder or case. Contact lens solution and the lenses *per se* are placed in the contact lens holder/case when it is used by a contact lens wearer and the device of the invention is able to detect whether or not the solution and/or lens introduces a microbial contamination into the holder/case.

By way of further example a device, or devices, according to the present invention may be incorporated within a wound dressing and used to detect microbial infection of wounds.

According to a second aspect of the invention there is provided a contact lens case characterised in that each chamber for retaining a lens comprises a device according to the first aspect of the invention.

It is preferred that each chamber of the contact lens holder comprises a hole in the bottom of the chamber. The hole may define a pore of a device according to the invention which is an integral part of the case. Alternatively a separate device according to the invention may be fixed to the case in order that the hole and the pore align to allow micro-organisms to enter the pore.

The pore, according to the seond aspect of the invention, may define a total volume of less than 100µl. In a preferred embodiment the pore has a volume of about 25 µl and may contain 20µl of reporting means and 5µl of a containment layer.

A physical barrier (e.g. a mesh made from PMMA with a mesh pore size of approximately 100µm) may be fixed above the pore in the bottom of the case chamber in "integral" device designs. Alternatively the physical barrier may be fixed above the pore in a recess in the housing that has been adapted to receive it in independent devices that are for affixing to the lens case.

The reporting means in devices according to the second aspect of the invention preferably comprise agar and a tetrazolium salt. It is most preferred that the agar is made up as about 1% w/v agar in Wilkins Chalgren broth. Wilkins Chalgren broth was found to be highly suitable for incorporation in a contact lens case (or any other medical device in which the reporting means will be immersed or covered by a liquid). The Wilkins Chalgren Agar may then be mixed with MTT or MTS at a concentration of indicator of about 500µg/ml to form the reporting means. A most preferred device according to the second aspect of the invention is described in Example 4.

According to a third aspect of the invention there is provided a wound dressing case characterised in that the dressing comprises at least one device according to the first aspect of the invention.

Preferred devices according to the third aspect of the invention may comprise independent units that may comprise a moulded plastic house containing a pore. The pores in such devices may be 2x2mm and in a preferred embodiment have 1x1mm pores.

Most preferred reporting means for use in devices according to the third aspect of the invention comprise a solution of 0.7% agarose in 1x Mueller Hinton Broth containing 500µg/ml MTT. When the pore is 1x1mm the volume of the pore in devices according to the third aspect is about 1.6µl and may contain about 1.4 µl of a reporting means and about 0.2µl of a liquid containment layer (e.g. 5% carboxymethyl cellulose). A mesh may also be fitted above the containment layer to retain the reporting means in the pore.

Devices according to the third aspect of the invention may be fitted into wound dressing by a number of ways. For instance individual devices may be woven into the fabric of the dressing and/or affixed by adhesive.

A number of devices may be fitted into the dressing such that there is an array of devices in the dressing.

Activation of the devices within the dressing will result in the production of dark dots in the dressing and this will indicate to a clinician or a user of the dressing that there is microbial contamination of at least the dressing and possible also the wound itself.

In a fourth aspect, the invention provides a method of analysing a sample for the presence of microorganisms, said method comprising the steps of:
(a) contacting the device provided by the first aspect of this invention with a sample to be analysed; and
(b) examining the reporting means to determine whether or not microorganisms are present in the sample.

In one embodiment, the method provided by the fourth aspect of this invention involves contacting the sample to be analysed with the sampling surface of the device.

In one embodiment, a sample may be applied directly to the sampling surface - perhaps using, for example a dispensing device such as a pipette. In other embodiments, the device and sample may be incubated together.

In a further embodiment, the device and sample are incubated together for a period of time and under conditions suitable to facilitate, encourage or cause any microorganisms present in the sample to pass to the device. Thereafter, the device may be incubated (with or without the sample) for a further period of time and under conditions suitable to facilitate, encourage or cause the passage of microorganisms into and through the pores defined by the sampling surface, towards the reporting means.

In one embodiment, the fourth aspect of this invention provides a method of analysing a sample for the presence of microorganisms, said method comprising the steps of:
(a) contacting a device provided by the first aspect of this invention with a sample to be analysed; and
(b) incubating the sample and device together for a period of time and under conditions suitable to facilitate the passage of any microorganisms present in the sample to the device and into and through the pores defined in the sampling surface thereof;
(c) incubating the device, with or without the sample, for a period of time and under conditions suitable to facilitate the passage of any microorganisms towards and into contact with, the reporting means; and
(d) examining the reporting means to determine whether or not microorganisms are present in the sample.

In use, the device or sampling surface thereof, may be brought into contact with a sample to be analysed and incubated for a period of time therewith. In this way, any microorganisms present in the sample migrate to, or colonise, the device and ultimately move into one or more of the pores defined by the sampling surface. Microorganisms that have successfully passed into a pore may then begin to multiply or move through the pore, towards the reporting means. At this point, the device may be left in contact with the sample to allow the reporting means to complete any reactions necessary to report the presence of microorganisms to the user. Alternatively, after a period of incubation with the sample, the device and sample could be separated and the device incubated for a further period of time before the reporting means is examined.

In one embodiment, the device may further comprise a detachable lid which can be removed to expose the sampling surface for contact with a sample and replaced to protect the sampling surface from contamination during periods when the device is incubated without the sample. A detachable lid may further protect the device from desiccation during periods of storage prior to use.

In one embodiment, the device provided by this invention (or methods described herein) may be used to identify, detect and/or quantify microorganisms present in a wound. In applications of this type, the sampling surface of the device is brought into contact with the wound and left *in situ* under conditions suitable to permit passage of any microorganisms present in the wound to the sampling surface.

Alternatively, a swab of the surface of a wound may be used to inoculate the sampling surface of the device - this avoids the need to directly contact the device with the wound.

Thereafter, microorganisms that have passed to the sampling surface may grow or move into a pore defined therein. The microorganisms may continue to migrate, multiply or grow towards the reporting means. After a suitable period of incubation (either with or without the wound), the device can be removed and the reporting means examined to determine whether or not any microorganisms were present in the wound.

In one embodiment, the device may comprise a heat source such that it can be incubated at a predetermined temperature without need for an incubator. Additionally, or alternatively, the device may further comprise a heat block, which can be used to maintain the device at a predetermined temperature.

In other embodiment, the device provided by the first aspect of this invention or method according to the fourth aspect, may be used to probe or analyse samples of water, soil, air and/or organic material (for example animal or plant matter) for the presence of microorganisms.

In a fifth aspect, the invention provides a kit for detecting microorganisms in a sample, the kit comprising a device according to the first or second aspects of this invention and one or more of the following:
(i) means for obtaining samples to be analysed; and
(ii) reagents and/or buffers for preparing, diluting and/or storing samples to be analysed.

In one embodiment, the means for obtaining samples may comprise a swab, scraper, loop or other tool for sampling.

According to a sixth aspect of the invention there is provided a use of a device as defined in the first aspect of the invention for detecting microorganisms in a sample or on an object, surface or other device.

The sample, object, surface or other device may be anything contemplated in the embodiments discussed above. For instance one preferred use of devices according to the invention is for monitoring the microbiological status of haemodialysis and peritoneal dialysis equipment (including water purification systems, dialyzers, catheters, fistulas and arteriovenous graft).

In one aspect, the invention is substantially described in the description and figures.

### DETAILED DESCRIPTION

The present invention will now be described in detail with reference to the following figures which show:
Figure 1 shows a cross-sectional view of a device according to one aspect of this invention.
Figure 2 shows the device of Figure 1 being used to probe a sample for the presence of microorganisms.
Figure 3: The MicroSensor device, as manufactured for use within a range of medical devices. The generic structure of the device is shown (A) including the effect of microbial entry leading to the device being triggered (B). This entire unit is designed for separate manufacture, and incorporation into a range of medical devices. The appearance of a prototype of this device is shown in (C). The effect of treatment with 10⁶ CFU *Pseudomonas aeruginosa* followed by overnight incubation is shown in (D).
Figure 4: Schematic representations of possible manufacturing processes. (A) continuous processing - hot embossing: (B) continuous processing - hot embossing (including backing): (C)Screen Printing onto laser ablated substrate: (D)Gravure screen printing allowing greater precision in region 4.
Figure 5: Typical appearance of the Microsensor Reporting Means after overnight incubation following microbial challenge.
Figure 6 A-C: A High Sensitivity Microsensor.
Figure 7 A-C: A Low Sensitivity Microsensor
Figure 8 is a photograph of 25 devices according to the second aspect of the invention with growth chamber dimensions of 2 x 2mm showing triggering of the reporting means in the first two rows.
Figure 9 is a photograph of 18 devices according to the second aspect of the invention with growth chamber dimensions of 4 x 2mm showing triggering of the reporting means in the first two rows.
Figure 10 represents photographs of: (A) a worn wound dressing; (B) a simulated control wound dressing (Smith and Nephew Allevyn Lite non adhesive dressing) moistened with PBS; and (C) 4 devices with growth chambers of 1x1mm containing growth media and MTT with triggering of the reporting means for the bottom two devices.
Figure 11: represents a schematic drawing of: (A) components of a lens case and a device according to the second aspect of the inventon; and (B) an assembled lens case incorporating a device according to the invention.
Figure 12: represents cross-sections of the lens and device depicted in Figure 12 wherein (A) is an expanded view of the components of the case and device; (B) illustrates the device during assembly; and (C) shows a completed lens case incorporating a device according to the invention.

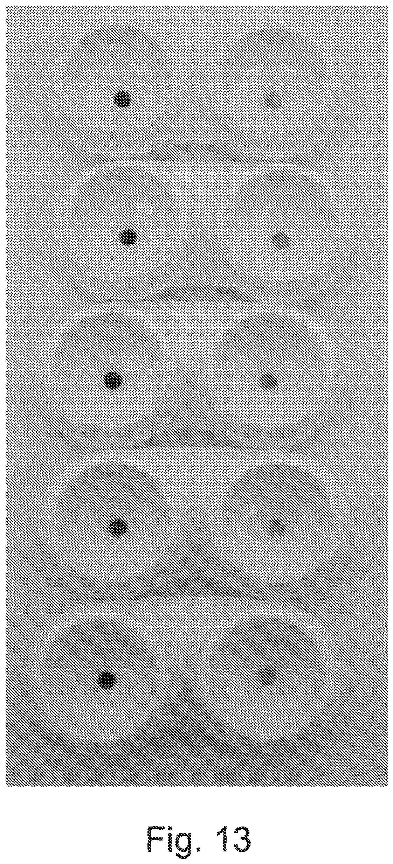

Figure 13: is a photograph of five contact lens cases which have a 4x2mm Microsensor device at the base of each well according to the second aspect of the invention. The left handside of each case exhibits triggering of the reporting means.

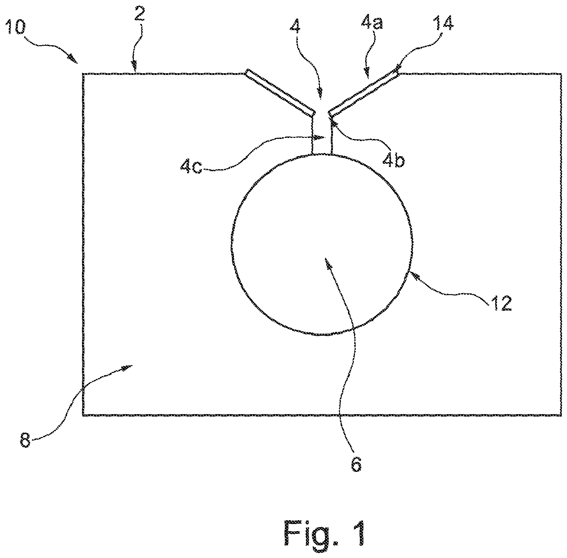

Turning to Figure 1, there is shown a device (10) for detecting, identifying and/or quantifying microorganisms in a sample, said device comprising a surface (2: herein after referred to as the sampling surface) for contact with a sample to be analysed (sample not shown here), said surface (2) defining a pore (4), said pore (4) comprising means (6) for reporting the presence of a microorganism.

In this embodiment, the sampling surface (2) is the upper surface of a substrate (8) of the device (10). The pore (4) defined by the sampling surface (2) comprises a wide opening (4a) at the sampling surface and a narrow opening (4b) below the sampling surface (2: i.e. countersunk therein). As can be seen in Figure 1, this arrangement forms a dimple or depression in the sampling surface which tapers from the narrow pore opening (4b) to the wide pore opening (4a) to serve as a funnel guiding material from the sampling surface (2) down into the pore (4) and ultimately through the pore shaft (4c). The sides of the funnel formed between wide pore opening (4a) and narrow pore opening (4b) are, in this embodiment coated with functionalising factors (14) which enhance, promote or support microbial colonisation, adherence, binding and/or growth .

The pore extends from the sampling surface (2) down, through the substrate 8 and terminating in a chamber (12) which contains the means for reporting microorganisms (6)

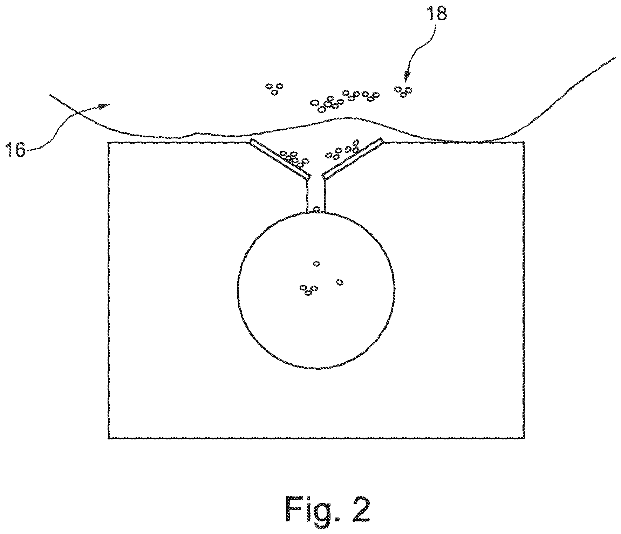

Figure 2 shows the device presented in Figure 1 in use. Here, a sample 16 which comprises microorganism (18) has been brought into contact with the sampling surface (2) of the device (10). The presence of functionalising factors (14) around the opening (or mouth) of the pore (4), encourages migration of the microorganisms (18) from the sample (16) to the sampling surface (2) where they colonise and grow around the pore openings (4a and 4b). As the microorganisms (18) grow or continue to migrate, they move down the pore (4: through the pore shaft (4c)) toward the reporting means (6) contained within the chamber (12) at the end of the pore (4). Ultimately, the microorganism reaches the reporting means (6) which, in this embodiment, comprises an indicator which undergoes a chemical reaction to produce a pigment when metabolised by a particular microorganism. As such, if the user detects the presence of a dye or pigment in the reporter means (6), he/she can conclude that the sample (16) contained microorganisms (18).

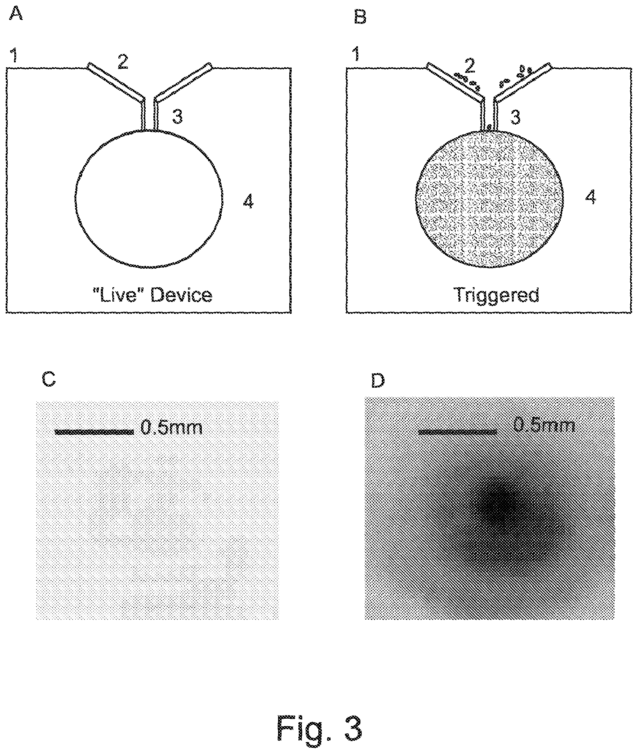

Figure 3: A shows the microsensor device prior to use and activation. Where 1) represents the outside of the microsensor surface, 2) represents the graduated opening towards the entry pore, 3) represents the channel leading to the growth chamber, and 4) represents the "inactivated" growth chamber which contains agar, nutrient broth, and inactive dye MTT.

Figure 3B shows the device in use, where 1) represents the outside of the microsensor surface, for instance in contact with a wound, 2) represents the graduated pore area with bacteria clearly present, 3) represents the channel with bacteria travelling towards the growth chamber, 4) represents the blue triggered growth chamber with a positive result for the presence of microorganisms/bacteria indicated with, in this case, MTT.

Figure 3C represents the visible appearance of an inactivated growth chamber containing agar, nutrient broth and inactive MTT, no bacteria are present.

Figure 3D represents the visible appearance of contamination, the blue coloured response as a result of the activation of MTT in response to the presence of microorganisms/bacteria.

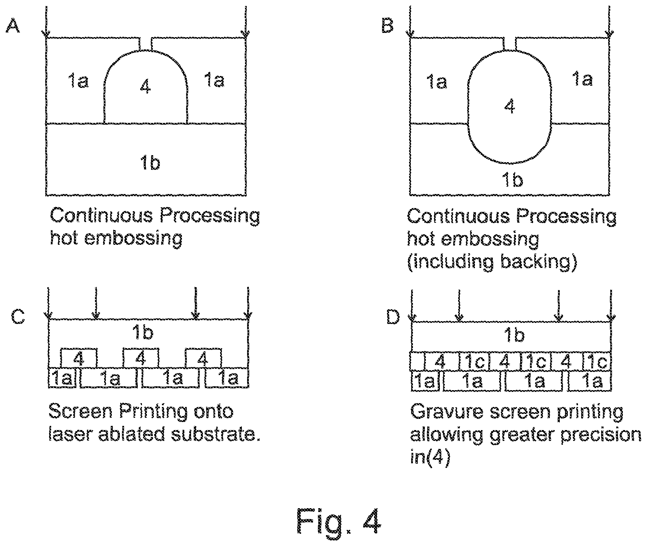

Figure 4 shows four possible manufacturing processes - each is discussed in more detail below:
Figure 4A: Continuous processing using a thin film polymeric substrate (labelled in Figure 4 as 1a). This substrate may be hot embossed to introduce hemispherical cavities into which an agar-based reagent (Figure 4 - labelled 4) is introduced. The cavity shapes may be varied, but one of skill will appreciate that sharp edges and corners should be avoided - the continuous roll hot embossing process will provide better mould tool release if the features are more "rounded". The cavities may be filled after the hot embossing of the substrate through either robotic dispensing, or a more simplistic planar filling, using, for example, a squeegee. The final processing stage may comprise the lamination of a backing film (see Figure 4 - labelled 1b). This could be incorporated using full or patterned coating of adhesive. Alternatively, continuous ultrasonic welding could join a backing film of identical polymer to the embossed substrate. The pore opening defined by 1a (in this case an opening of about 1µm) may be created using laser ablation of the substrate polymer after the hot embossing step.
   b) Continuous processing, similar to that described in a) above, but a hot embossing process has been used to generate a spherical cavity in a backing film (1b).
   c) Screen printing is used to introduce precise volumes of an agar reagent onto a predrilled polymer substrate. This could be a continuous roll process, but it may be necessary to use gravure screen printing to ensure a more precise agar reagent shape and volume. The agar could then be frozen, within a continuous roll process, and a backing material (labelled 1b) added as a liquid film. This film may be solidified by a variety of techniques, including UV-curing etc.
   d) A further application of a screen printing technique, but this time the geometric structure to contain the agar reagent is first formed by gravure screen printing (labelled 1c). Subsequently, the agar reagent is introduced using either robotic dispensing, or planar filling with, for example, a squeegee. Freezing of the agar, would then allow a similar encapsulation, as described in c).

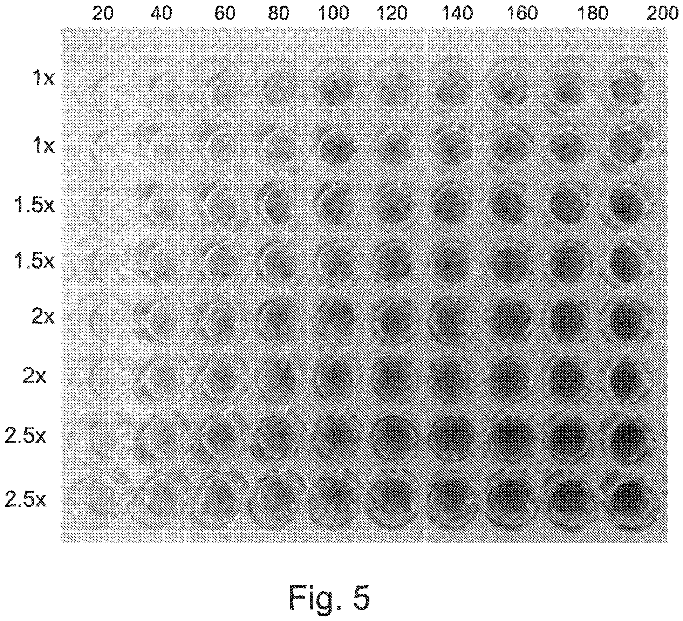

Figure 5 shows the results of challenging typical microsensor reporting means, consisting of broth in agar with metabolic indicator, using various levels of broth and MTT, after challenge with *S. aureus.*

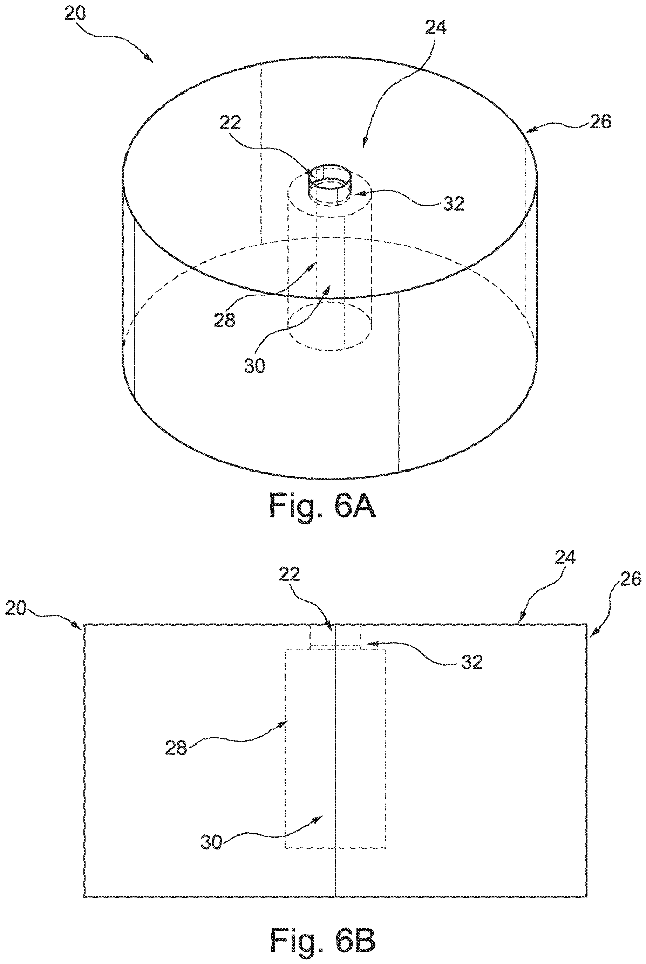

Figures 6A and B show a single microsensor unit (20) as might be found in a highly sensitive microsensor device. In this embodiment, the microsensor unit 20 comprises a pore 22 defined within the sampling surface 24 of a substrate 26. The pore 22 is 500µm wide and extends 250µm into the substrate 26. Pore 22 connects to a chamber 28 containing the reporting means 30 which, in this embodiment, comprises 0.3% (W/v) agar, 1.5x Mueller-Hinton broth and MTT at 100µg/ml. The chamber 28 is cylindrical and about 1mm in diameter and extends about 2mm into the substrate 24 (in other words the chamber is about 2mm deep). Between the pore 22 and the chamber 28 containing the reporting means 30, is provided a barrier or containment layer (32). This layer 32 prevents desiccation and egress of components of the reporting means 30 from the chamber 28 and is about 50µm deep.

One of skill will appreciate that since the sampling surface 24 of the substrate 26 defines a wide/short pore 22, the sensitivity of the device is high as low numbers of microorganisms can readily and quickly find their way into the chamber 28 containing the reporting means 30 to activate the device.

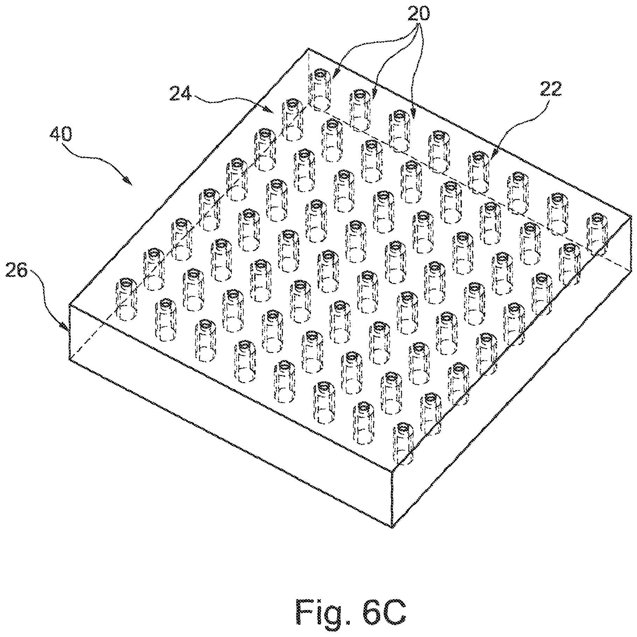

Figure 6C shows an array of microsensor units 20 in a microsensor device 40. In this embodiment, the device 40 comprises 8x8 microsensor units. In this embodiment, the distance between the central point (i.e. the centre of each pore 22 defined by the surface 24 of the substrate 26) of each microsensor unit 20 from the central point of a neighbouring microsensor unit 20, is about 2.5mm.

It should be appreciated that the density of microsensor units 20 in the device greatly increases the ability of the device 40 to detect the presence of microorganisms in a sample.

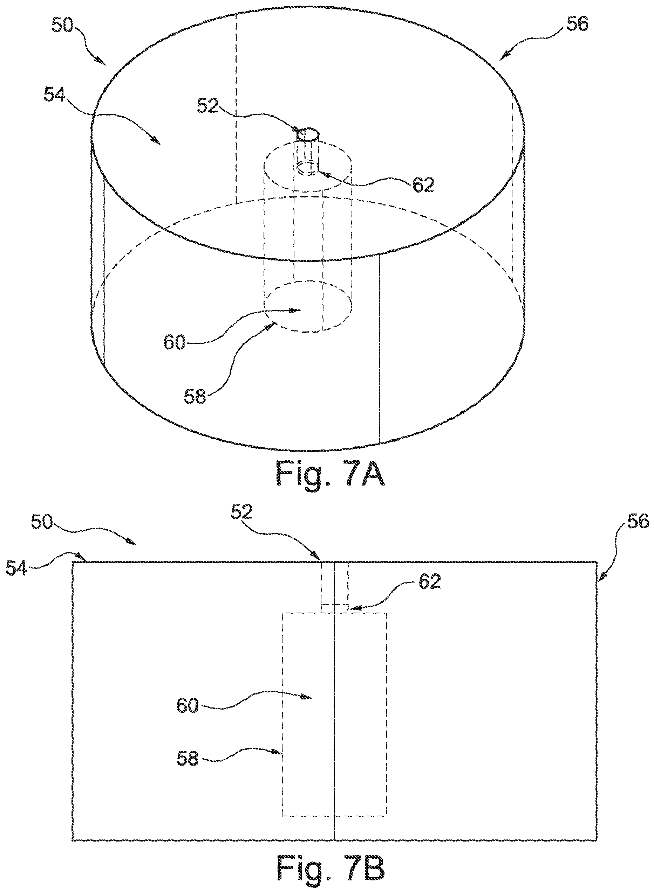

Figures 7A and B show a single microsensor unit (50) as might be found in a low sensitivity microsensor device. In this embodiment, the microsensor unit 50 comprises a pore 52 defined within the sampling surface 54 of a substrate 56. The pore 52 is 250µm wide and extends 500µm into the substrate 56. Pore 52 connects to a chamber 58 containing the reporting means 60 which, in this embodiment, comprises 0.3% (W/v) agar, 1.5x Mueller-Hinton broth and MTT at 100µg/ml. The chamber 58 is cylindrical and about 1mm in diameter and extends about 2mm into the substrate 54 (in other words the chamber is about 2mm deep). Between the pore 52 and the chamber 58 containing the reporting means 60, is provided a barrier or containment layer (62). This layer 62 prevents desiccation and egress of components of the reporting means 60 from the chamber 58 and is about 50µm deep.

One of skill will appreciate that since the sampling surface 54 of the substrate 56 defines a narrow/long pore 52, the sensitivity of the device is low as it is more difficult for microorganisms to find their way into the chamber 58 containing the reporting means 60 to activate the device. In this way, only samples with high levels of microbial contamination may activate the device.

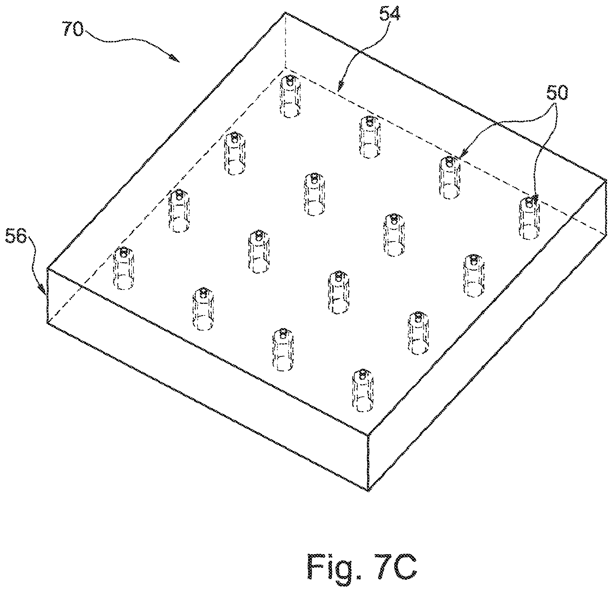

Figure 7C shows an array of microsensor units 50 in a microsensor device 70. In this embodiment, the device 70 comprises 4x4 microsensor units 50. In this embodiment, the distance between the central point (i.e. the centre of each pore 52 defined by the surface 54 of the substrate 56) of each microsensor unit 50 from the central point of a neighbouring microsensor unit 50, is about 2.5mm.

It should be appreciated that the low density of microsensor units 50 in the device 70 decreases the sensitivity.

Figure 8 is a photograph of 5x5 prototype microsensor units 80. Each unit had 2x2mm cylindrical pores and are discussed in more detail in Example 2.

Figure 9 is a photograph of 3x6 prototype microsensor units 90. Each unit 4x2mm cylindrical pores and are discussed in more detail in Example 2.

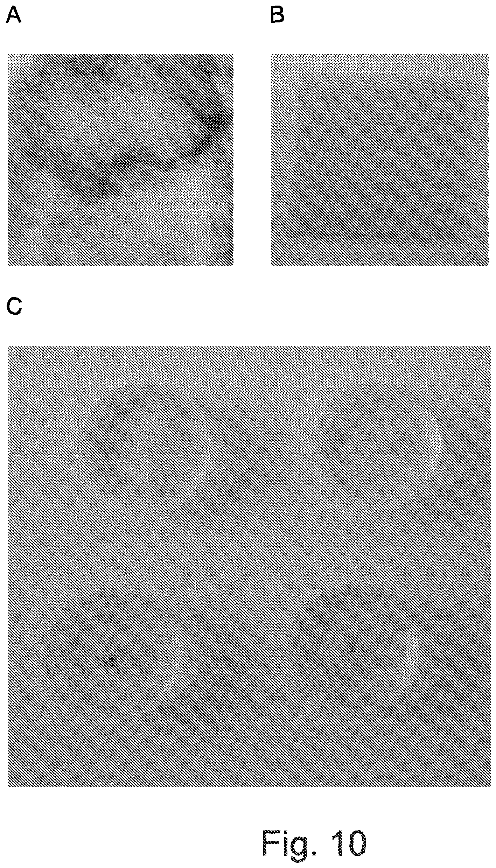

Figure 10 is a photograph of 4x4 prototype microsensor units 100 that may be suitable for incorporating into wound dressings. Each unit had pores with 1x1mm openings and are discussed in more detail in Example 3.

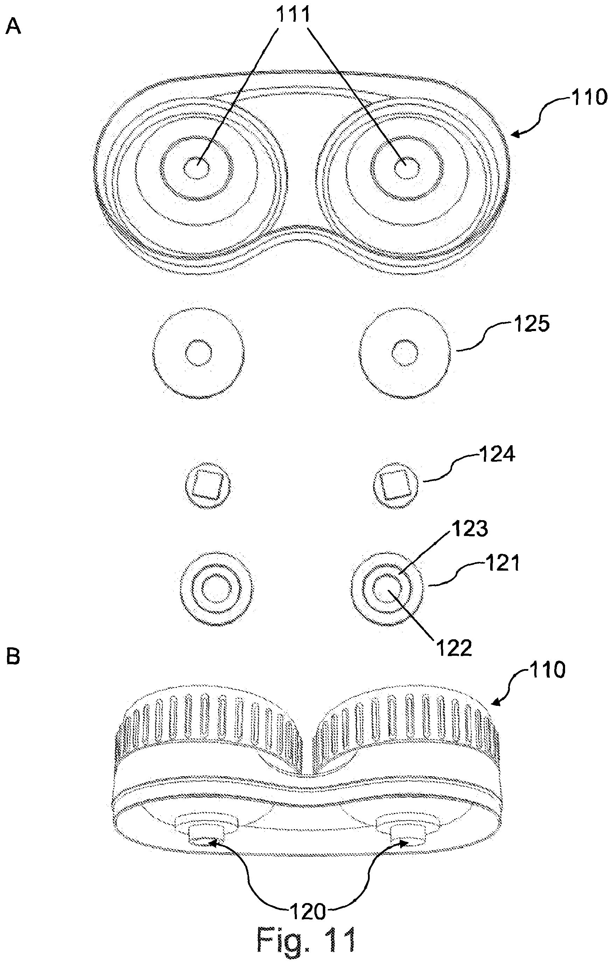

Figures 11A and B show a contact lens case (110) which has been adapted to incorporate microsensor units (120) in holes (111) formed in the left and right chambers of the case. Microsensor units (120) for joining to the case (110) comprise a sensor housing (121) made out of polypropylene in which a pore (122) and recess (123) are machined in the surface of the housing. The pore (122) is adapted to receive approximately 25µl in volume and the recess (123) defines an area above the pore (122) which is adapted to fit a mesh (124, a barrier layer according to the invention). The mesh may typically be a PMMA mesh disk with 100µm mesh pores that, in use, allows bacteria to enter the pore but prevent the reporting means being detached from the pore. The case (110) and device (120) are assembled using a suitable adhesive (125) that does not obstruct communication between the pore (122) and the chambers of the case (110). The adhesive (125) may be an acrylic adhesive such as 3M double sided acrylic adhesive 468MP 200MP.

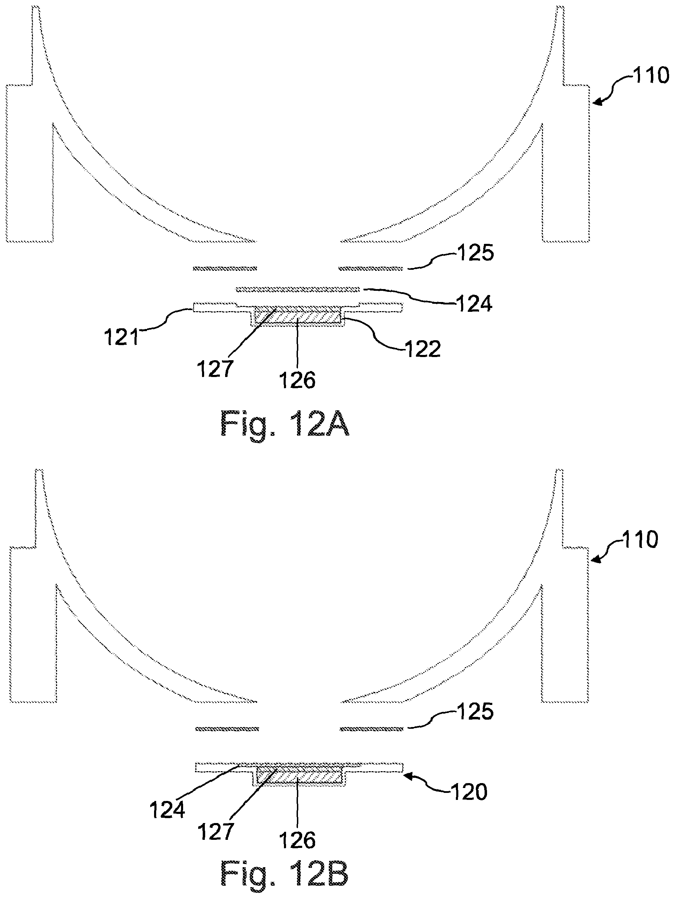

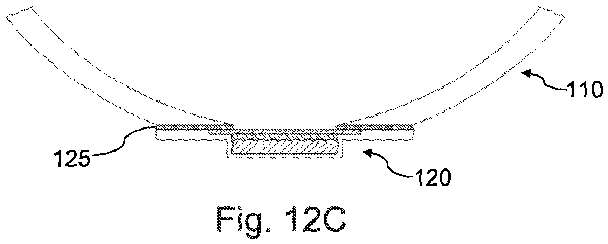

Figure 12 represents cross-sections of the contact lens case (110) and a microsensor unit (120) as illustrated in Figure 11. Figure 12A as an expanded illustration of the contact lens case (110), the sensor housing (121), mesh (124) and adhesive (125). The sensor housing (121) as illustrated has a pore (122) which has been filed with first a reporting means (126) and then a containment layer (127) above it. The reporting means is typically agar made up with a nutrient broth and further comprising a tetrazolium salt as an indicator. The containment layer (127) may comprise a viscous liquid (e.g 5% carboxymethyl cellulose in PBS) which allows bacteria to enter the microsensor unit (120) while at the same time maintaining the viability of the reporting means before use (e.g. when the assembled case is in storage). Figure 12B illustrates the device during assembly wherein the mesh (124) is placed in the recess (123) of the sensor housing (121). Figure 12C illustrates an assembled case (110) and microsensor (120). It should be noted that the adhesive (125) fixes the case (110) and microsensor (120) together and that mesh (124) and recess for retaining it (123) are dimensioned such that the adhesive (125) also holds the mesh (124) in place.

### EXAMPLE 1

The inventor realised that there were no commercially available products that were small and simple for detecting microbial contamination of samples or objects. Initial experiments were therefore conducted to evaluate whether or not a detectable signal of microbial contamination could be generated in a simple miniaturised system.

### 1.1 Materials & methods

**1.1.1.** Initial experiments evaluated whether a disk of agar containing MTT which was encased in a polymer could develop a visible blue signal when exposed to a bacteria.
   Drops of tryptone soya broth containing 0.5% (w/v) agar were placed on a petri dish and allowed to set. Silicone was poured over the droplets and set at 80°C for 1 hour. Once set, disks were cut out using a cork borer. 2µL 5mg/mL MTT was added to the agar and allowed to soak in. The silicone agar disks impregnated with MTT were placed in a petri dish. *Pseudomonas aeruginosa* from an overnight culture was re-suspended and diluted to ∼10⁹cfu/mL in PBS. 1µL of suspension (containing ∼10⁶cfu P*seudomona aeruginosa*) was exposed to the disks and incubated at 37°C overnight in a humidity chamber. Controls were exposed to 1µL sterile PBS.
**1.1.2.** Following the work conducted in 1.1.1 the inventors decided to evaluate whether or not a positive signal could be achieved in varying concentrations of agar.
   Mueller-Hinton broth was prepared containing 1%, 0.7%, 0.5% and 0.3% (w/v) agar, added to the wells of duplicate 24-well microtitre plates and allowed to set in air. Overnight suspensions of microbial cultures (*Staphylococcus aureus, Staphylococcus epidermidis,* EMRSA, *Pseudomonas aeruginosa, Escherichia coli, Bacillus cereus, Enterococcus faecalis, Klebsiella pneumoniae, Serratia marcescens* and *Candida albicans*) were diluted to 10⁷cfu/mL and 500µL added to the agars. Plates were incubated overnight at 37°C and room temperature respectively.
**1.1.3.** Further work examined whether or not variations in the concentration of broth and indicator (MTT) would affect signal generation.
   Mueller-Hinton broth at 1x, 1.5x, 2x and 2.5x normal strength, was prepared with 0.3 % (w/v) agar. MTT solubilised in distilled water was sterilised and added to the sloppy agar to give final concentrations ranging 20 to 200 µg/mL in 20µg/mL increments. The range of agars were added in a chequerboard style, with broth concentration increasing with each row, and MTT increasing with each column, to the wells of a 96-well microtitre plate and allowed to set in air. Overnight suspensions of microbial cultures (*Staphylococcus aureus, Pseudomonas aeruginosa* and *Candida albicans*) were diluted to 10⁷cfu/mL and 50µL added to the agars. Plates were incubated overnight at room temperature.

### 1.2 Results

**1.2.1** Figure 3D represents the typical appearance of triggered disks made according to method 1.1.1 after overnight incubation following microbial challenge with *Pseudomonas aeruginosa.* Figure 3C represents the typical appearance of silicone disks containing agar and MTT, but not exposed to *Pseudomonas aeruginosa,* and incubated in the same petri dish as the exposed sensors remained colourless.
**1.2.2** Each of the agar concentrations tested as outlined in 1.1.2 sustained micro-organisms overnight and resulted in the triggering of MTT such there was a colour change from translucent yellow to strong blue (observed for all microorganisms grown at both 37°C or room temperature) (data not shown)
**1.2.3** Figure 5 shows the data generated following the protocol outlined at 1.1.3 above for *Staphylococcus aureus.* Agar colour change from translucent yellow to blue was observed for all agar/MTT combinations, with intensity increasing with each broth and MTT concentration increase. Similar data were obtained for the other organisms.

### 1.3 Discussion

These data made the inventors realise that it would be possible to generate simple "Microsensor" devices that are capable of reliably triggering following challenge with a microbial suspension. Triggering of the indicator resulted in an unequivocal dark purple visual signal appearing overnight after challenge, without any need for operator intervention. Such a signal can be easily recognised by an observer, who is made aware that the sensor has come into contact with an abnormally high level of microbes.

### EXAMPLE 2:

Having established that reporting means (Example 1) could be triggered by micro-organisms of interest, the inventors proceeded to make prototype devices according to the invention and tested whether or not such devices could be selectively and sensitively triggered by micro-organisms of interest.

### 2.1 Materials & methods

### 2.1.1 Prototype devices

**2.1.1.1** The prototype devices illustrated in Figures 8 were formed by the following steps:
   (a) A device housing was manufactured by injection moulding using crystal polystyrene. The mould defined the housing, surface of the device and a 2x2mm cylindrical pore (diameter x depth). The overall dimensions of the devices were 10mmx10mmx3mm. The device was then sterilised.
   (b) making a solution of 0.7% agarose in 1x Mueller Hinton Broth. This was then autoclaved. After cooling (to 40°C), a sterile filtered stock solution of MTT is added to a concentration of 500µg/ml to form the reporting means.
   (c) 6µl of the reporting means was then added to the pore of the device using a Hamilton Syringe and allowed to solidify for an hour.
**2.1.1.2** The prototype devices illustrated in Figures 9 were formed by the following steps:
   (a) A device housing was manufactured by injection moulding using crystal polystyrene. The mould defined the housing, surface of the device and a 4x2mm cylindrical pore (diameter x depth). The overall dimension of the device were 10mmx10mmx3mm. The device was then sterilised.
   (b) making up Wilkins Chalgren agar (1% agar). This was then autoclaved. After cooling (to 40°C), a sterile filtered stock solution of MTT is added to a concentration of 500µg/ml to form the reporting means.
   (c) 25µl of the reporting means was then added to the pore of the device using a Hamilton Syringe and allowed to solidify for an hour.

### 2.1.2 Protocols for testing triggering

**2.1.2.1:** Testing of Devices with 2x2mm cylindrical pores.
   Stock suspensions of *Pseudomonas aeruginosa* ATCC9027 were made as 10⁹, 10⁸, 10⁷, 10⁶ and 10⁵ cfu/ml stocks. 1µl of each stock was inoculated onto the top of the pore of a device to give 10⁶, 10⁵, 10⁴, 10³ or 10² cells on each device.
   The devices were then placed in an incubator and left overnight at 30°C. The colour change was observed the next day.
**2.1.2.1:** Testing of Devices with 4x2mm cylindrical pores.
   Stock suspensions with an optical density (570nm) of one were made up for *Staphylococcus aureus* ATCC 6538 (10⁸ cfu/mL); *Pseudomonas aeruginosa* ATCC9027(10⁸ cfu/mL); *Candida albicans* ATCC 10231(10⁷ cfu/mL) *Serratia marcesens* ATCC 13880 (10⁸ cfu/mL) and *Fusarium solani* ATCC 36031.
   1ml of each 1OD stock was inoculated onto the top of device retained in a 24 well plate. The plate was then left overnight at room temperature and the colour change observed the next day.

### 2.2 Results

### 2.2.1 Sensing of a range of micro-organisms

Figure 8 is a photograph of 25 devices (prepared according to 2.1.1.1) with growth chamber dimensions of 2 x 2mm which had been inoculated with *Pseudomonas aeruginosa* ATCC9027 or control solution (no micro-organism) according to 2.1.2.1. After a 1µL inoculation of each device, the top row of devices shows reproducible triggering with a dose of 10⁶ cfu (top row) or 10⁵ cfu (second row) of *Pseudomonas aeruginosa.* The third and fourth rows show no triggering at 10⁴ and 10³ cfu, with the fifth row representing a phosphate buffered saline control. This data confirms the 2x2mm devices containing growth media and MTT are reproducible with respect to both triggering and cfu sensitivity. Similar results were obtained with *Staphylococcus aureus, Candida albicans, Serratia marcesens,* and *Fusarium solani* (data not shown).

### 2.2.2 Sensing of a micro-organisms associated with ocular infection

Figure 9 is a photograph of 18 devices with pore dimensions of 4 x 2mm (prepared according to 2.1.1.2) which had been inoculated with micro-organisms or control solution according to 2.1.2.2). The photograph shows triggering of the reporting means (in triplicate) with the five ISO standard contact lens organisms. From left to right the first column represents a phosphate buffered saline control, the second *Staphylococcus aureus* ATCC 6538 10⁸ cfu/mL, third *Pseudomonas aeruginosa* ATCC 9027 10⁸ cfu/mL, fourth *Candida albicans* ATCC 10231 10⁷ cfu/mL, fifth *Serratia marcesens* ATCC 13880 10⁸ cfu/mL, sixth *Fusarium solani* ATCC 36031 (1 OD) overnight culture re-suspended in PBS. This data demonstrates that the 4x2 mm devices are capable of triggering reproducibly in the presence of typical organisms associated with ocular infection.

### 2.3 Discussion

These data illustrated that prototype devices according to the invention were able to detect micro-organisms in a reproducible way and in particular illustrated that devices according to the invention may be useful in the management of ocular infection (e.g. devices used according to the third aspect of the invention).

### EXAMPLE 3:

The inventors proceeded to test devices according to the the invention in the context of a real situation by evaluating the usefulness of the devices for detecting microbial contamination of a wound dressing.

### 3.1 Materials & methods

### 3.1.1 Moulding of Device housing

Device housings were manufactured by a third party from PMMA with SU8 epoxy by moulding with 1x1mm pores (a volume of approximately 1.6 µl). A silicon coating (PDMS) was applied to the surface to make it easier to identify the surface of the device containing the opening to the pore.

### 3.1.2 Filling the device housing

(a) A solution of 0.7% agarose in 1x Mueller Hinton Broth was made. This was then autoclaved. After cooling (to 40°C), a sterile filtered stock solution of MTT was added to a concentration of 500µg/ml to form the reporting means.
(b) 1.4µl of the reporting means was then added to the pore of the device using a Hamilton Syringe and allowed to solidify for an hour. 0.2 µl of 5% carboxycellulose (in PBS) was placed on top of the reporting means as a containment layer

### 3.1.3 Wound Dressings

A wound dressing (Smith and Nephew Allevyn Lite non adhesive dressing) worn by a patient with a microbial infection was obtained with the consent of the patient. Figure 10A is a photograph of the worn wound dressing.

A clean/unused dressing (Smith and Nephew Allevyn Lite non adhesive dressing) moistened with sterile PBS was used as a control. Figure 10B is a photograph of the simulated control wound dressing.

### 3.1.4 Protocols for testing contamination of a wound dressing

Devices were placed in a petri dish and either a worn dressing or unused dressing pressed on top. The dish and dressing were left over night at 30°C and any colour change observed the next day.

### 3.2 Results

Figure 10C is a photograph of four devices according to the invention. The top two devices were placed into contact with the control dressing and show no triggering of the indicator whereas the lower two devices were placed in contact with the worn dressing and demonstrated a positive signal in both cases. This confirms that devices according to the invention are capable of detecting infection within a clinical environment.

### 3.3 Discussion

These data illustrate that devices according to the invention are capable of detecting infection within a wound environment. A skilled person will appreciate that the devices may be adapted such that they may be incorporated as an array of microsensors within the fabric of a wound dressing. In use, a dressing may be lifted from a wound and inspected. Activation of devices (dark dots within the pores of the devices) will indicate that an infection is present in the wound area and such knowledge may be used to direct future actions. For instance a decision may be taken to at least change the dressing. A clinician may also wish to consider the extent to which the wound is infected and may wish to initiate a course of antibiotics.

### EXAMPLE 4:

The data presented at 2.2.2 inspired the inventors to develop devices according to the invention that may be incorporated into contact lens cases. The inventors realised that devices according to the invention may be used to inform a user that the case and/or the solution within it and/or lenses *per se* placed in the case have been contaminated by micro-organisms. The user may then decide whether or not to discard the solution; discard or clean the lenses; and/or to discard the lens case as appropriate and thereby reduce the risk of developing an eye infection by introducing a contact lens into an eye which has come from a contaminated lens.

Contact lens cases according to the third aspect of the invention were made by the following procedures:

### 4.1. Manufacture of a housing for the device

A device housing was manufactured by injection moulding using either white or crystal polystyrene. The inventors found that a white housing allowed a user to better observe a colour change from inside the chamber of a contact lens case whereas crystal polystyrene allowed a colour change for observing from outside (from below) the case.

A 4x2mm cylindrical pore (diameter x depth) with a volume of approximately 25µl was provided in the moulding. A recess (for receiving a mesh) was provided in the surface of the housing containing the opening to the pore. The device housing was then sterilised.

### 4.2 Preparation of a Reporting Means

Wilkins Chalgren agar (1% agar) was made up and then autoclaved. After cooling (to 40°C), a sterile filtered stock solution of MTT was added to a concentration of 500µg/ml to form the reporting means.

### 4.3 Charging the pore of the device

20µl of the reporting means was then added to the pore of the device using a Hamilton Syringe and allowed to solidify for an hour. 5µl of 5% carboxycellulose (in PBS) was then placed on top of the reporting means as a containment layer in the top of the pore.

### 4.4. Fitting a Physical barrier

A PMMA mesh disk with 100µm mesh was then placed in the recess provided with in the moulded housing.

### 4.5 Assembly with a Contact Lens Case

Contact lens cases were obtained and 4mm diameter holes drilled in the bottom of the case chamber which receive lens solution and a lens when in use. If the type of case required it, the base of the contact lens case was machined and flattened to improve adherence of the device.

3M double sided acrylic adhesive 468MP 200MP was the affixed to the underside of each lens case chamber and the lens case careful placed on top of the device according to the inventions (such that the pores and holes align) and allowed to adhere thereto.

The devices are further illustrated in Figures 11 and 12 and further described in the specific description above.

### EXAMPLE 5:

The inventors proceeded to test the devices of Example 5 to evaluate whether or not the reporting means is triggered by inoculation of the chambers of the contact lens case with organisms associated with ocular infection.

### 5.1 Materials & methods

### 5.1.1 Devices

Devices were made as described in Example 4.

### 5.1.2 Protocols for testing contact lens cases incorporating microsensor devices

Stock suspensions with an optical density (570nm) of one were made up for *Staphylococcus aureus* ATCC 6538 (10⁸ cfu/mL); *Pseudomonas aeruginosa* ATCC9027(10⁸ cfu/mL); *Candida albicans* ATCC 10231(10⁷ cfu/mL) *Serratia marcesens* ATCC 13880 (10⁸ cfu/mL) and *Fusarium solani* ATCC 36031.

1ml of each 1OD stock was inoculated into the lens holding chambers of the contact lens case. The case was then left overnight at room temperature and the colour change observed the next day.

### 5.2 Results

Figure 14 is a photograph of five contact lens cases which each have a device according to the invention at the base of each lens chamber/well.

The right hand chamber of each of the lens cases was exposed to a control phosphate buffered saline solution. The devices were not triggered and retained their negative yellow colour. The left hand side of each lens case was exposed to contact lens ISO organism strains from top to bottom *S.aureus* ATCC 6538 10⁸ cfu/mL, *P.aeruginosa,* ATCC 9027 10⁸ cfu/mL, *C.albicans* ATCC 10231 10⁷ cfu/mL, *S.marcesens* ATCC 13880 10⁸ cfu/mL , *F.solani* ATCC 36031 overnight culture and then re-suspended in PBS. Each device demonstrates a clearly visible positive result demonstrating the suitable application of the device in detecting contact lens related infections.

### 5.3 Discussion

These data clearly show that devices according to the invention are particularly useful for detecting microbial contamination of contact lens cases. The devices described in Example 4, and variants thereof, may be cheaply and easily mass produced and are of great utility to people who wear contact lenses and wish to minimise the risk of developing an eye infection.

## Claims

1. A microorganism detecting device, said device comprising a support or body component formed and adapted to have a surface for contact with a sample to be analysed, said surface defining a pore, said pore comprising means for reporting the presence of a microorganism **characterised in that** the reporting means comprises:
a solid or semi-solid substrate;
a tetrazolium salt; and
a nutrient broth selected from Mueller Hinton Broth or Wilkins Chalgren Broth
wherein the pore defines a volume of less than 500µl and wherein the device comprises a physical barrier that allows microorganisms to enter the reporting means but retains the components of the reporting means within the pore.

2. The device according to claim 1 wherein the tetrazolium salt is MTT (3-(4,5-Dimethylthiazol-2-yl)-2,5-diphenyltetrazolium bromide) or MTS (3-(4,5-dimethylthiazol-2-yl)-5-(3-carboxymethoxyphenyl)-2-(4-sulfophenyl)-2H-tetrazolium).

3. The device according to claim 1 or 2 wherein the solid or semi-solid substrate is agar or agarose.

4. The device according to claim 3 wherein the substrate is agar and the reporting means comprises about 1 % w/v agar or the substrate is agarose and the reporting means comprises about 0.7 % w/v agarose.

5. The device according to any preceding claim wherein the pore is cylindrical pore and has a diameter of 10mm.

6. The device according to any preceding claim further comprising a containment layer located between where the sample will be located when in use and the reporting means in the pore.

7. The device according to claim 6 wherein in the containment layer comprises a viscous liquid approximately 100 - 400 µm thick or deep.

8. The device according to any preceding claim wherein the physical barrier is a mesh.

9. A contact lens case **characterised in that** each chamber for retaining a lens comprises a device according to any one of claims 1 - 8.

10. A wound dressing **characterised in that** the dressing comprises at least one device according to any one of claims 1 - 8.

11. A method of analysing a sample for the presence of microorganisms, said method comprising the steps of:
(a) contacting a device according to any one of claims 1 - 8 with a sample to be analysed; and
(b) examining the reporting means to determine whether or not microorganisms are present in the sample.

12. A use of a device according to any one of claims 1-8 for detecting microorganisms in a sample or on an object, surface or other device.

## Patentansprüche

1. Mikroorganismus-Nachweisvorrichtung, wobei die Vorrichtung eine Träger- oder Körperkomponente umfasst, die dafür gestaltet und ausgelegt ist, eine Oberfläche für den Kontakt mit einer zu analysierenden Probe aufzuweisen, wobei die Oberfläche eine Pore definiert, wobei die Pore Einrichtungen zum Melden des Vorhandenseins eines Mikroorganismus aufweist, **dadurch gekennzeichnet, dass** die Meldungsvorrichtung umfasst:
ein festes oder halbfestes Substrat;
ein Tetrazoliumsalz; und
eine Nährbrühe ausgewählt aus Mueller-Hinton-Brühe und Wilkins-Chalgren-Brühe,
wobei die Pore ein Volumen von weniger als 500 µl definiert und wobei die Vorrichtung eine physikalische Barriere umfasst, die das Eintreten von Mikroorganismen in die Meldungsvorrichtung erlaubt aber die Komponenten der Meldungsvorrichtung in der Pore zurückhält.

2. Vorrichtung gemäß Anspruch 1, wobei das Tetrazoliumsalz MTT (3-(4,5-Dimethylthiazol-2-yl)-2,5-diphenyltetrazoliumbromid) oder MTS (3-(4,5-Dimethylthiazol-2-yl)-5-(3-carboxymethoxyphenyl)-2-(4-sulfophenyl)-2H-tetrazolium) ist.

3. Vorrichtung gemäß Anspruch 1 oder 2, wobei das feste oder halbfeste Substrat Agar oder Agarose ist.

4. Vorrichtung gemäß Anspruch 3, wobei das Substrat Agar ist und die Meldungsvorrichtung etwa 1 % Gew./Vol. Agar umfasst oder das Substrat Agarose ist und die Meldungsvorrichtung etwa 0,7 % Gew./Vol. Agarose umfasst.

5. Vorrichtung gemäß einem der vorstehenden Ansprüche, wobei die Pore eine zylindrische Pore ist und einen Durchmesser von 10 mm aufweist.

6. Vorrichtung gemäß einem der vorstehenden Ansprüche, ferner umfassend eine Eingrenzungsschicht, die zwischen dem Ort, an dem in Verwendung die Probe angeordnet sein wird, und der Meldungsvorrichtung in der Pore angeordnet ist.

7. Vorrichtung gemäß Anspruch 6, wobei die Eingrenzungsschicht eine viskose Flüssigkeit umfasst, die etwa 100-400 µm dick oder tief ist.

8. Vorrichtung gemäß einem der vorstehenden Ansprüche, wobei die physikalische Barriere in Netz ist.

9. Kontaktlinsenbehälter, **dadurch gekennzeichnet, dass** jede Kammer zum Enthalten einer Linse eine Vorrichtung gemäß einem der Ansprüche 1-8 umfasst.

10. Wundverband, **dadurch gekennzeichnet, dass** der Verband wenigstens eine Vorrichtung gemäß einem der Ansprüche 1-8 umfasst.

11. Verfahren zum Analysieren einer Probe auf das Vorhandensein von Mikroorganismen, wobei das Verfahren die Schritte umfasst:
(a) Inkontaktbringen einer Vorrichtung gemäß einem der Ansprüche 1-8 mit einer zu analysierenden Probe; und
(b) Untersuchen der Meldungsvorrichtung, um zu bestimmen, ob Mikroorganismen in der Probe vorhanden sind oder nicht.

12. Verwendung einer Vorrichtung gemäß einem der Ansprüche 1-8 zum Nachweisen von Mikroorganismen in einer Probe oder auf einem Gegenstand, einer Oberfläche oder einer anderen Vorrichtung.

## Revendications

1. Dispositif de détection de micro-organisme, ledit dispositif comprenant un composant de support ou de corps formé et adapté pour comporter une surface pour contact avec un échantillon à analyser, ladite surface définissant un pore, ledit pore comprenant un moyen pour rapporter la présence d'un micro-organisme **caractérisé en ce que** le moyen de révélation comprend :
un substrat solide ou semi-solide ; un sel de tétrazolium ; et
un bouillon nutritif choisi parmi le bouillon de Mueller-Hinton ou le bouillon de Wilkins-Chalgren dans lequel le pore définit un volume inférieur à 500 µl et le dispositif comprenant une barrière physique qui permet à des micro-organismes d'entrer dans le moyen de révélation mais retient les composants du moyen de révélation dans le pore.

2. Dispositif selon la revendication 1 dans lequel le sel de tétrazolium est MTT (bromure de 3-(4,5-diméthylthiazol-2-yl)-2,5-diphényltétrazolium) ou MTS (3-(4,5-diméthylthiazol-2-yl)-5-(3-carboxyméthoxyphényl)-2-(4-sulfophényl)-2H-tétrazolium).

3. Dispositif selon la revendication 1 ou 2 dans lequel le substrat solide ou semi-solide est la gélose ou l'agarose.

4. Dispositif selon la revendication 3 dans lequel le substrat est la gélose et le moyen de révélation comprend environ 1 % m/v de gélose ou le substrat est l'agarose et le moyen de révélation comprend environ 0,7 % m/v d'agarose.

5. Dispositif selon l'une quelconque des revendications précédentes dans lequel le pore est un pore cylindrique et a un diamètre de 10 mm.

6. Dispositif selon l'une quelconque des revendications précédentes comprenant en outre une couche de confinement située entre l'emplacement où l'échantillon sera situé en cours d'utilisation et le moyen de révélation dans le pore.

7. Dispositif selon la revendication 6 dans lequel la couche de confinement comprend un liquide visqueux d'approximativement 100 à 400 µm d'épaisseur ou de profondeur.

8. Dispositif selon l'une quelconque des revendications précédentes dans lequel la barrière physique est un treillis.

9. Lentille de contact **caractérisée en ce que** chaque chambre pour retenir une lentille comprend un dispositif selon l'une quelconque des revendications 1 à 8.

10. Pansement de plaie **caractérisé en ce que** le pansement comprend au moins un dispositif selon l'une quelconque des revendications 1 à 8.

11. Procédé d'analyse d'un échantillon pour la présence de micro-organismes, ledit procédé comprenant les étapes de :
(a) mise en contact d'un dispositif selon l'une quelconque des revendications 1 à 8 avec un échantillon devant être analysé ; et
(b) examen du moyen de révélation pour déterminer si des micro-organismes sont présents ou non dans l'échantillon.

12. Utilisation d'un dispositif selon l'une quelconque des revendications 1 à 8 pour détecter des micro-organismes dans un échantillon ou sur un objet, une surface ou un autre dispositif.
